# EUROPEAN PATENT SPECIFICATION

(11) **EP 3 643 302 B1**
(45) Date of publication and mention of the grant of the patent: **20.09.2023**
(21) Application number: 18820786.4
(22) Date of filing: 22.06.2018
(51) Int. Cl.: A61K 31/01, A61K 31/336, A61K 31/365, A23L 33/10, A61K 8/31, A23K 20/10, A61P 21/00, A61Q 19/00

(54) **COMPOSITION CONTAINING SESQUITERPENE DERIVATIVE AS ACTIVE INGREDIENT FOR PREVENTION OR TREATMENT OF MUSCLE DISEASES**
ZUSAMMENSETZUNG MIT SESQUITERPENDERIVAT ALS WIRKSTOFF ZUR PRÄVENTION ODER BEHANDLUNG VON MUSKELERKRANKUNGEN
COMPOSITION COMPRENANT UN DÉRIVÉ DE SESQUITERPÈNE EN TANT QUE PRINCIPE ACTIF POUR LA PRÉVENTION OU LE TRAITEMENT DE MALADIES MUSCULAIRES

(30) Priority: 23.06.2017 KR 20170079813
(43) Date of publication of application: 29.04.2020
(73) Proprietor: Industry-Academic Cooperation Foundation Yonsei University, Seoul 03722 (KR)
(72) Inventor: PARK, Tae Sun, Seoul 03174 (KR)
(74) Representative: Petty, Catrin Helen
(86) International application number: PCT/KR2018/007104
(87) International publication number: WO 2018/236186

(56) References cited:
- WO-A1-2008/144880
- CN-A- 103 641 841
- KR-A- 20150 138 963
- KR-B1- 100 905 419
- KR-B1- 101 092 620
- US-A1- 2010 292 306
- US-A1- 2015 246 087
- US-A1- 2016 089 340
- YANG QUANJUN ET AL: "Parthenolide from Parthenium integrifolium reduces tumor burden and alleviate cachexia symptoms in the murine CT-26 model of colorectal carcinoma", PHYTOMEDICINE (JENA), vol. 20, no. 11, August 2013 (2013-08), pages 992-998, XP002799125, ISSN: 0944-7113
- ELMANN, A. et al.: "Glutamate Toxicity to Differentiated Neuroblastoma N2a Cells Is Prevented by the Sesquiterpene Lactone Achillolide A and the Flavonoid 3,5,4'-trihydroxy -6,7,3'-trimethoxyflavone from Achillea Fragrantissima", Journal of Molecular Neuroscience, vol. 62, no. 1 11 April 2017 (2017-04-11), pages 99-105, XP036234599, [retrieved on 2017-04-11]
- DATABASE BIOSIS [Online] BIOSCIENCES INFORMATION SERVICE, PHILADELPHIA, PA, US; August 2013 (2013-08), YANG QUANJUN ET AL: "Parthenolide from Parthenium integrifolium reduces tumor burden and alleviate cachexia symptoms in the murine CT-26 model of colorectal carcinoma", Database accession no. PREV201300649651 & PHYTOMEDICINE (JENA), vol. 20, no. 11, August 2013 (2013-08), pages 992-998, ISSN: 0944-7113, DOI: 10.1016/J.PHYMED.2013.04.020

## Description

### TECHNICAL FIELD

This application claims priority to and the benefit of Korean Patent Application No. 10-2017-0079813, filed on June 23, 2017.

The present invention is defined in the appended claims. It relates to a pharmaceutical composition for use in preventing or treating a muscle disease, which comprises, as an active ingredient, sesquiterpene derivatives or pharmaceutically acceptable salts thereof.

### BACKGROUND ART

In 2000, the elderly population in South Korea accounted for 7.2% of the total population and thus Korea has entered an aging society, and is expected to enter a super-aging society in 2050 (2013 elderly statistics, the National Statistical Office). Muscle mass in humans decreases with age (about 10-15% at an age of 50-70 years, and 30% or more decrease at an age of 70-80 years), and accordingly, muscle strength and muscle function are also weakened, which is referred to as age-related sarcopenia. Age-related sarcopenia is a major cause of limiting the independent living of the elderly by inducing activity disorders and gait disturbances. In addition, sarcopenia lowers a basal metabolic rate, increases insulin resistance, promotes type 2 diabetes, and increases the risk of hypertension and cardiovascular disease by 3-5 times. Currently, no drug has been approved for the treatment of sarcopenia, and drug repositioning technology is being developed to apply a myostatin inhibitor or other FDA-approved agents for the treatment of diseases to sarcopenia.

Muscles are divided into skeletal muscles, cardiac muscles, and visceral muscles, and thereamong, skeletal muscles are the most abundant tissues in the human body, accounting for 40-45% of body weight. Skeletal muscles are attached to the bone by tendons, creating bone movement or force. One muscle is made up of numerous myofibers, which in turn are made up of numerous myofibrils consisting of actin and myosin. When actin and myosin move by overlapping each other, the length of muscles shortens or increases, causing overall muscle contraction and relaxation. An increase in myofibril size means an increase in myofiber thickness, resulting in an increase in muscle.

The type of myofibers that constitute muscles is mainly classified into Type I, Type IIA, and Type IIB by a metabolic process and a contraction rate that produce ATP. "Type I myofibers" have a low contraction rate and contain a large number of myoglobin and mitochondria, which are suitable for sustained, low-intensity aerobic activity. Type I myofibers have a red color and are also called red muscles, and the soleus is typical. Meanwhile, "Type IIB myofibers" are very short, but are used for high-intensity anaerobic exercise due to a high contraction rate thereof, and contain a low amount of myoglobin, thus having a white color. "Type IIA myofibers" have characteristics between the aforementioned two myofibers. With age, not only does the composition of Type I and II myofibers for each muscle site vary, but all types of myofibers are also reduced.

Skeletal muscles have the characteristics of being regenerated and maintained according to the environment, but these characteristics are lost with age, and consequently, as aging progresses, muscle mass is reduced and muscle strength is also lost. As a signaling system involved in muscle growth and regeneration, there is signaling which is mediated by insulin like growth factor 1 (IGF-1)/AKT to regulate protein synthesis. The activation of an IGF-1 receptor (IGF-1R) present in the muscle cell membrane increases AKT phosphorylation through IRS1 and PI3K phosphorylation, and the latter activates mTORC phosphorylation. The activation of mTORC increases the phosphorylation of ribosomal protein S6 kinase beta-1 (p70S6K1), thereby not only increasing mRNA translation, but also increasing the activity of eukaryotic translation initiation factor 4 G (eIF4G) and phosphorylating eukaryotic translation initiation factor 4E binding protein 1 (4E-BP1). eIF4G and 4E-BP1 are involved in the formation of an eIF4F complex. That is, eIF4G binds to eIF4A and eIF4E to form an eIF4F complex, while phosphorylation of 4E-BP1 inhibits binding capacity for eIF4E, leading to an increase in free eIF4E. The latter combines with other translation initiation factors (eIF4G and eIF4A) to form an eIF4F complex, which in turn promotes translation initiation by stabilizing ribosomal structures, ultimately increasing protein synthesis (Bodine et al., Akt/mTOR pathway is a crucial regulator of skeletal muscle hypertrophy and can prevent muscle atrophy in vivo. Nature Cell Biology, 3, 1014-1019, 2001).

In addition, AKT phosphorylation stimulates myofiber growth by increasing eIF2B expression through glycogen synthase kinase 3 (GSK3) and also inhibits muscle loss by inhibiting the expression of forkhead box O (FOXO), which is a proteolytic transcription factor. Muscle loss is regulated by signaling mediated by receptors of the TGF-family, including myostatin, transforming growth factor beta (TGF-β), and activin. The binding of a ligand to TGF-β type II receptor phosphorylates a Type I receptor, and the latter phosphorylates the smad 2/3 complex and eventually activates FOXO. The latter increases the gene expression of muscle-specific ubiquitin-ligases, i.e., muscle RING-finger protein-1 (MURF1) and muscle atrophy F-Box (MAFbx)/atrogin-1, which attach ubiquitin to the lysine site of a target protein to promote proteolysis, eventually leading to muscle loss (Gumucio et al., Atrogin-1, MuRF-1, and sarcopenia. Endocrine, 43, 12-21, 2013).

### DESCRIPTION OF EMBODIMENTS

### TECHNICAL PROBLEM

An object of the present invention is to provide a pharmaceutical composition for use in the prevention or alleviation of a muscle disease, muscle differentiation promotion, muscle regeneration, muscle function improvement, or muscle strengthening, the pharmaceutical composition comprising, as an active ingredient, a sesquiterpene derivative or a pharmaceutically acceptable salt thereof.

Another object of the present invention is to provide a health functional food composition or livestock feed composition for use in the prevention or alleviation of a muscle disease, muscle differentiation promotion, muscle regeneration, muscle function improvement, or muscle strengthening, the composition comprising a sesquiterpene derivative or a pharmaceutically acceptable salt thereof as an active ingredient.

Another object of the present invention is to provide the claimed compositions for use in a method of preventing or treating a muscle disease, promoting muscle differentiation, regenerating muscles, or strengthening muscles, the method comprising administering, to a subject, a pharmaceutical composition comprising a sesquiterpene derivative or a salt thereof as an active ingredient, or allowing it to be taken.

Another object of the present invention is to provide the claimed composition for use in the prevention or treatment of a muscle disease, muscle differentiation promotion, muscle regeneration, or muscle strengthening, the composition comprising a sesquiterpene derivative or a salt thereof as an active ingredient.

However, technical problems to be solved by the present invention are not limited to the above-described technical problems, and other unmentioned technical problems will become apparent from the following description to those of ordinary skill in the art.

### TECHNICAL SOLUTION

According to an aspect of the present invention, there is provided a pharmaceutical composition for use in preventing or treating a muscle disease, comprising a sesquiterpene derivative or a pharmaceutically acceptable salt thereof as an active ingredient.

According to one embodiment of the present invention, the sesquiterpene derivative may be any one or more selected from compounds represented by Formulae 1 to 15.

According to an exemplary embodiment of the present invention, the sesquiterpene derivative may be α- cedrene.

According to one embodiment of the present invention, the composition may increase the expression of the p-4E-BP1 protein and the p-p70S6K protein.

According to one embodiment of the present invention, the composition may reduce the gene expression of muscle RING-finger protein-1 (MuRF1), muscle atrophy F-box (MaFbx), or myostatin.

According to one embodiment of the present invention, the muscle disease may be a muscle disease caused by muscle dysfunction, muscle loss, muscle atrophy, muscle wasting, or muscle degeneration.

According to an exemplary embodiment of the present invention, the muscle disease may be any one or more selected from the group consisting of atony, muscular atrophy, muscular dystrophy, myasthenia, cachexia, rigid spine syndrome, amyotrophic lateral sclerosis (Lou Gehrig's disease), Charcot-Marie-Tooth disease, and sarcopenia.

An embodiment of the present invention also provides a pharmaceutical composition for use in muscle differentiation promotion, muscle regeneration, muscle function improvement, or muscle strengthening, comprising a sesquiterpene derivative or a pharmaceutically acceptable salt thereof as an active ingredient.

Another embodiment of the present invention also provides a health functional food composition for use in preventing or alleviating a muscle disease, promoting muscle differentiation, regenerating muscles, improving muscle function, or strengthening muscles, comprising a sesquiterpene derivative or a pharmaceutically acceptable salt thereof as an active ingredient.

Another embodiment of the present invention also provides a livestock feed composition for use in preventing or alleviating a muscle disease, promoting muscle differentiation, regenerating muscles, improving muscle function, or strengthening muscles, comprising a sesquiterpene derivative or a pharmaceutically acceptable salt thereof as an active ingredient.

Another embodiment of the present invention also provides the claimed composition for use in a method of preventing or treating a muscle disease, promoting muscle differentiation, regenerating muscles, or strengthening muscles, comprising administering, to a subject, a pharmaceutical composition comprising a sesquiterpene derivative or a pharmaceutically acceptable salt thereof as an active ingredient, or allowing it to be taken.

Another embodiment of the present invention provides the claimed compositions for use in preventing or treating a muscle disease, promoting muscle differentiation, regenerating muscles, or strengthening muscles, the composition comprising a sesquiterpene derivative or a pharmaceutically acceptable salt thereof as an active ingredient.

### ADVANTAGEOUS EFFECTS OF INVENTION

The present invention relates to a composition for use in preventing or treating a muscle disease, or improving muscle function, comprising a sesquiterpene derivative or a pharmaceutically acceptable salt thereof as an active ingredient, and the sesquiterpene derivative can increase the expression of proteins related to myoprotein synthesis and a muscle mass increase in muscle cells, and can inhibit the expression of a myoprotein degradation-related enzyme at an mRNA level. Thus, the composition can exhibit a muscle strengthening effect through muscle differentiation, muscle regeneration, and a muscle mass increase in muscle diseases caused by muscle dysfunction, muscle loss, or muscle degeneration, and can inhibit muscle loss, and thus can be used for the prevention or treatment of a muscle disease, muscle differentiation, muscle regeneration, muscle strengthening, a muscle mass increase, myogenesis promotion, or muscle function improvement.

### BRIEF DESCRIPTION OF DRAWINGS

FIG. 1 illustrates changes in thickness of myotubes in mouse myoblasts treated with cedrene (mean ± standard error of three independent experiments, p < 0.05).
FIG. 2 illustrates changes in thickness of myotubes in mouse myoblasts treated with cedrenol (A), methyl cedryl ketone (B), cedrene epoxide (C), β-cedrene (D), cedryl acetate E, or cedrol (F).
FIG. 3 illustrates changes in expression of proteolysis- and protein synthesis-related molecules in mouse myoblasts treated with cedrene.
FIG. 4 illustrates the results of confirming increases in grip strength of mice by intake of cedrene.
FIG. 5 illustrates the results of confirming increases in limb muscle strength of mice by intake of cedrene.
FIG. 6 illustrates the results of confirming changes in fiber diameter of muscle tissues of mice by intake of cedrene (mean ± standard error of eight mice, p < 0.05).

### BEST MODE

The inventors of the present invention confirmed that sesquiterpene derivatives inhibited the degradation of myoproteins and promoted the synthesis thereof, and thus are effective in strengthening muscles and alleviating muscle loss, thus completing the present invention.

The term "muscle" as used herein collectively refers to tendon, muscle, and sinew, and "muscle function" means the ability of muscle to exert a force through muscle contraction, and encompasses: muscle strength, which is the ability of muscle to exert maximum contraction to overcome resistance; muscular endurance, which is the ability to indicate how long or how many times muscle can repeat contraction and relaxation at a given weight; and explosive muscular strength, which is the ability to exert a strong force within a short period of time. Such muscle functions are proportional to muscle mass, and "muscle function improvement" means the act of making muscle functions better.

Hereinafter, the present invention will be described in detail.

### Pharmaceutical Composition for Prevention or Treatment of Muscle Disease

The present invention provides a pharmaceutical composition for use in the prevention or treatment of a muscle disease, comprising a sesquiterpene derivative or a pharmaceutically acceptable salt thereof as an active ingredient. In particular, the sesquiterpene derivative may be represented by Formula 1, 2, or 3 below: 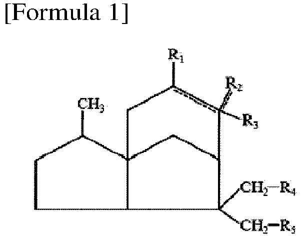 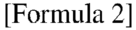 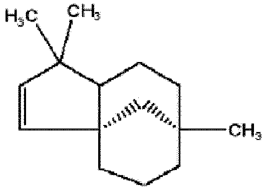 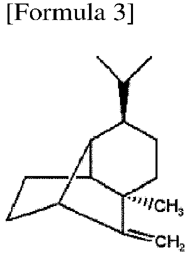 wherein, in Formulae 1 to 3, R₁ is hydrogen, hydroxyl, halo, a C₁-C₃ alkyl or -CO-R₆; R₆ is hydrogen or a C₁-C₃ alkyl; R₂ is hydrogen, hydroxyl, or a C₁-C₃ alkyl; R₁ and R₂ may be linked to each other to form an epoxide; R₃ is hydrogen, a C₁-C₃ alkyl, -O-CO-R₇, or a C₁-C₃ alkoxy; R₇ is hydrogen or a C₁-C₃ alkyl; R₄ is hydrogen, hydroxyl, or a C₁-C₃ alkoxy; R₅ is hydrogen, hydroxyl, or a C₁-C₃ alkoxy; -̅ -̅ -̅ -̅ -̅ -̅ -̅ -̅ denotes a single bond or a double bond; when -̅ -̅ -̅ -̅ -̅ -̅ -̅ -̅ in the ring structure is a double bond, R₂ is hydrogen, hydroxyl, or a C₁-C₃ alkyl; when -̅ -̅ -̅ -̅ -̅ -̅ at R₂ is a double bond, R₂ is CH₂; and when R₂ is a methyl group, R₃ is a hydroxyl group, and R₅ is hydrogen, R₄ is not hydrogen.

According to an exemplary embodiment of the present invention, R₁ is hydrogen, hydroxyl, or -CO-R₆; R₆ is a C₁-C₃ alkyl; R₂ is hydroxyl or a C₁-C₃ alkyl; R₁ and R₂ may be linked to each other to form an epoxide; R₃ is hydroxyl, a C₁-C₃ alkyl, -O-CO-R₇ or a C₁-C₃ alkoxy; R₇ is hydrogen or a C₁-C₃ alkyl; R₄ is hydrogen or hydroxyl; R₅ is hydrogen or hydroxyl; -̅ -̅ -̅ -̅ -̅ -̅ -̅ -̅ denotes a single bond or a double bond; when -̅ -̅ -̅ -̅ -̅ -̅ -̅ -̅ in the ring structure is a double bond, R₂ is a C₁-C₃ alkyl; when -̅ -̅ -̅ -̅ -̅ -̅ -̅ -̅ at R₂ is a double bond, R₂ is CH₂; and when R₂ is a methyl group, R₃ is a hydroxyl group, and R₅ is hydrogen, R₄ is not hydrogen.

According to an exemplary embodiment of the present invention, R₁ is hydrogen, hydroxyl, or -CO-CH₃; R₂ is hydroxyl or -CH₃; R₁ and R₂ may be linked to each other to form an epoxide; R₃ is hydroxyl, -CH₃, -O-CO-CH₃ or -OCH₃; R₄ is hydrogen or hydroxyl; R₅ is hydrogen or hydroxyl; -̅ -̅ -̅ -̅ -̅ -̅ -̅ -̅ denotes a single bond or a double bond; when -̅ -̅ -̅ -̅ -̅ -̅ -̅ -̅ in the ring structure is a double bond, R₂ is CH₃; when -̅ -̅ -̅ -̅ -̅ -̅ -̅ -̅ at R₂ is a double bond, R₂ is CH₂; and when R₂ is a methyl group, R₃ is a hydroxyl group, and R₅ is hydrogen, R₄ is not hydrogen.

In the present specification, "C₁-C₃ alkyl" refers to a linear or branched saturated hydrogen carbon group, and examples thereof include methyl, ethyl, n-propyl, and isopropyl. The term "halo" refers to halogen group elements, and examples thereof include fluoro, chloro, bromo, and iodo, preferably fluoro, chloro, or bromo. The term "alkoxy" refers to -O alkyl group. Substitution by a C₁-C₃-substituted alkyl group means substitution by a halo-, preferably chloro- or fluoro-, and more preferably a fluoro-substituted alkyl group. The term "epoxide" refers to a cyclic ether in which, in a single molecule, an oxygen atom binds to two carbon atoms to form a ring.

The sesquiterpene derivatives according to the present invention are included in an extract or fraction of a plant belonging to the genus Cupressus.

The plant belonging to the genus Cupressus refers to approximately 20 species of evergreen conifers, which belong to the family Cupressaceae, is used for ornamental wood, and is widespread in temperate and subtropical regions of Asia, Europe, and North America. Among plants belonging to other genera of the same family, especially many tall evergreen trees, which secrete and have the smell of resin, such as false cypress and cypress pine are also referred to as cypress. It grows up to 25 m in height and has a pyramid shape especially when young. The plant belonging to the genus Cupressus used in the present invention is not particularly limited as long as it includes a sesquiterpene derivative, and is preferably *Cupressus macnabiana* (Laurence G. Cool, Phytochemistry, 58(6): 969-972(2001)), *Cupressus nootkatensis* (Erdtman, H. et al., Acta Chem. Scand., 11:1157-1161(1957)), *Cupressus sempervirens* (Seyyed Ahmad Emami et al., Iranian Journal of Pharmaceutical Sciences, 2(2): 103-108(2006)), *Cupressus macrocarpa* (Srikrishna et al., Synthetic Communications, 37(17):2855-2860(2007)), *Cupressus bakeri* (Koon-Sin Ngo et al., J. Chem. Soc., Perkin Trans. 1, 189-194(2000), or *Cupressus funebris,* and is most preferably *Cupressus funebris.*

A Cupressus extract including a sesquiterpene derivative may be obtained from a plant belonging to the genus Cupressus (preferably, the xylem of a plant belonging to the genus Cupressus using a general extraction solvent, and may be obtained using, as an extraction solvent, preferably (a) a C₁-C₄ anhydrous or hydrous lower alcohol (e.g., methanol, ethanol, propanol, butanol, normal-propanol, isopropanol, normal-butanol, and the like, (b) a mixed solvent of the lower alcohol and water, (c) acetone, (d) ethyl acetate, (e) chloroform, (f) 1,3-butylene glycol, (g) hexane, (h) diethyl ether, (i) butyl acetate, or (i) water.

A Cupressus fraction including a sesquiterpene derivative refers to a more separated/purified form obtained by further separating/purifying the Cupressus extract. For example, the Cupressus fraction includes fractions obtained by allowing the Cupressus extract to pass through an ultrafiltration membrane having a certain molecular weight cut-off value, and fractions obtained through additionally performed various purification methods, such as separation by various types of chromatography (manufactured for separation according to size, charge, hydrophobicity, or affinity). The sesquiterpene derivative obtained through fractionation of the plant belonging to the genus Cupressus used in the present invention is preferably cedryl acetate represented by Formula 4 below, α-cedrene represented by Formula 5 below, β-cedrene represented by Formula 6 below, α-funebrene represented by Formula 7 below, or β-funebrene represented by Formula 8 below, and is more preferably cedryl acetate, α-cedrene, or β-cedrene.

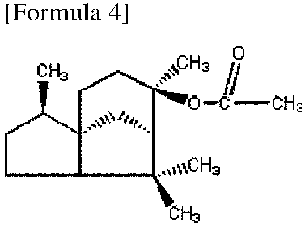

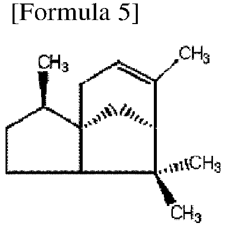

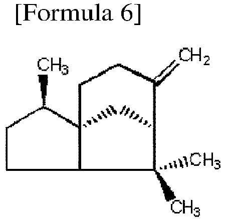

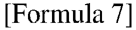

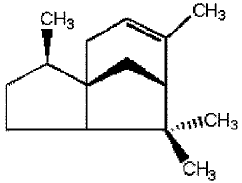

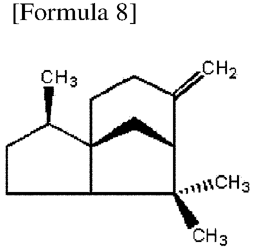

In addition, the sesquiterpene derivatives may be chemically synthesized.

According to an exemplary embodiment of the present invention, the sesquiterpene derivative includes various sesquiterpene derivatives prepared through synthesis other than separation from the plant belonging to the genus Cupressus. The sesquiterpene derivative more preferably includes cedrene epoxide represented by Formula 9 below, cedryl formate represented by Formula 10 below, methyl cedryl ether represented by Formula 11 below, 8(15)-cedrene-9-ol represented by Formula 12 below, epicedrol represented by Formula 13 below, methyl cedryl ketone represented by Formula 14 below, or cedrenol represented by Formula 15 below, and most preferably includes cedrene epoxide, methyl cedryl ether, methyl cedryl ketone, or cedrenol.

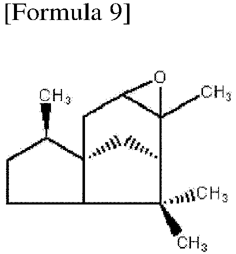

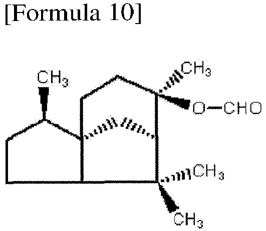

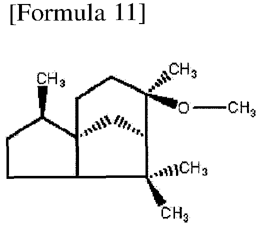

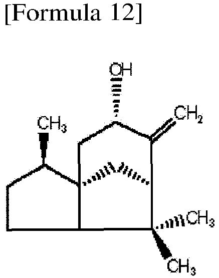

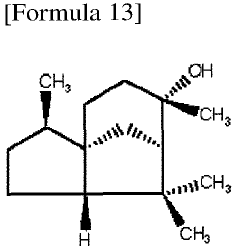

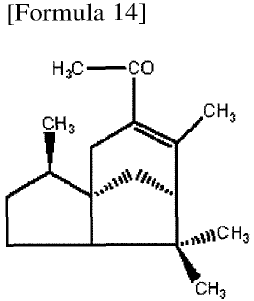

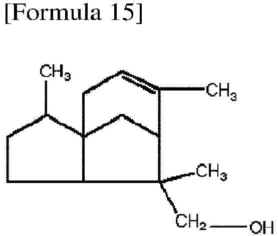

The composition according to the present invention may increase the expression of the p-4E-BP1 protein and the p-p70S6K protein, or may reduce the gene expression of muscle RING-finger protein-1 (MuRF1), muscle atrophy F-box (MaFbx), or myostatin. In particular, representative molecules related to protein synthesis include p70S6K1, 4E-BP1, and eIF members, and the activity of these three molecules is regulated by higher mTORCs. The activation of mTORc phosphorylates p70S6K1, and the activated p70S6K1 phosphorylates 40S ribosomal protein S6 to increase mRNA translation. In addition, the activation of mTORC not only increases the activity of eIF4G, but also phosphorylates 4E-BP1, and both molecules are involved in forming an eIF4F complex. That is, eIF4G binds to eIF4A and eIF4E to form an eIF4F complex, while, when 4E-BP1 is phosphorylated, the ability thereof to bind to eIF4E is inhibited, increasing eIF4E in a free state. The latter binds to other translation initiation factors (eIF4G and eIF4A) to form an eIF4F complex, which in turn promotes translation initiation by stabilizing the ribosomal structures, ultimately increasing protein synthesis. MAFbx/Atrogin-1 and MuRF1 are muscle-specific ubiquitin-ligases, which are representative proteins that promote proteolysis by attaching ubiquitin to the lysine site of a target protein, and induce muscle loss, and the composition according to the present invention may reduce the expression of muscle RING-finger protein-1 (MuRF1) or muscle atrophy F-box (MaFbx), thereby inhibiting muscle loss.

In the present invention, the "muscle disease" may be a muscle disease that is caused by muscle dysfunction, muscle loss, muscle atrophy, muscle wasting, or muscle degeneration and is reported in the art, and particularly, the muscle disease may be any one or more selected from the group consisting of atony, muscular atrophy, muscular dystrophy, myasthenia, cachexia, rigid spine syndrome, amyotrophic lateral sclerosis (Lou Gehrig's disease), Charcot-Marie-Tooth disease, and sarcopenia, but the present invention is not limited thereto. In addition, the muscle wasting or degeneration is caused by whole factors, acquired factors, aging, and the like, and muscle wasting is characterized by the gradual loss of muscle mass, and weakness and degeneration of muscles, especially skeletal or voluntary muscles and cardiac muscles.

The sesquiterpene derivative may be included at a concentration of 0.1 µM to 1,000 µM, but the present invention is not limited thereto. In this regard, when the concentration of the sesquiterpene derivative is less than the above range, protein synthesis and degradation activity in myocytes are reduced such that it is difficult to exhibit the effect of preventing or treating a muscle disease, and when the concentration of the sesquiterpene derivative is greater than the above range, there may be concerns about toxicity including cytotoxicity.

The pharmaceutical composition for use in preventing or treating a muscle disease according to the present invention may be formulated into the form of oral formulations such as powder, granules, tablets, capsules, a suspension, an emulsion, a syrup, and an aerosol, an agent for external applications, a suppository, and a sterile injection solution according to general methods, and may include suitable carriers, excipients, or diluents which are commonly used for the preparation of pharmaceutical compositions for formulation.

The carriers, excipients, or diluents may include various compounds or mixtures including lactose, dextrose, sucrose, sorbitol, mannitol, xylitol, erythritol, maltitol, starch, acacia gum, alginates, gelatin, calcium phosphate, calcium silicate, cellulose, methyl cellulose, micro-crystalline cellulose, polyvinylpyrrolidone, water, methylhydroxy benzoate, propylhydroxy benzoate, talc, magnesium stearate, and mineral oil.

When the pharmaceutical composition is formulated, a commonly used diluent or excipient such as a filler, a thickener, a binder, a wetting agent, a disintegrating agent, a surfactant, or the like may be used.

Solid preparations for oral administration may be prepared by mixing a bean extract with at least one of excipients, for example, starch, calcium carbonate, sucrose or lactose, gelatin, or the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance agent, a preservative, and the like.

Preparations for parenteral administration include an aqueous sterile solution, a non-aqueous solvent, a suspension, an emulsion, a freeze-dried preparation, and a suppository. Non-limiting examples of the non-aqueous solvent and the suspension solvent include propylene glycol, polyethylene glycol, a vegetable oil such as olive oil, and an injectable ester such as ethyl oleate. Examples of suppository bases include Witepsol, Macrogol, Tween 61, cacao butter, laurin, glycerol, gelatin, and the like.

A suitable dose of the pharmaceutical composition for the prevention or treatment of a muscle disease according to the present invention varies according to conditions and body weight of patients, the severity of disease, drug form, administration route, and administration period, and may be appropriately selected by those of ordinary skill in the art. However, for desired effects, the pharmaceutical composition may be administered in an amount of 0.0001 mg/kg/day to 2,000 mg/kg/day, preferably 0.001 mg/kg/day to 2,000 mg/kg/day. The pharmaceutical composition may be administered in a single dose or multiple doses daily. However, the dose is not intended to limit the scope of the present invention.

The pharmaceutical composition for the prevention or treatment of a muscle disease according to the present invention may be administered to mammals such as mice, rats, livestock, humans, or the like via various routes. All modes of administration may include, for example, oral injection, rectal or intravenous injection, muscular injection, subcutaneous injection, intrauterine epidural injection, and intracerebroventricular injection.

### Pharmaceutical Composition for Muscle Differentiation Promotion, Muscle Regeneration, or Muscle Strengthening

The present invention provides a pharmaceutical composition for use in muscle differentiation promotion, muscle regeneration, or muscle strengthening, which includes a sesquiterpene derivative or a pharmaceutically acceptable salt thereof as an active ingredient. The detailed description of the sesquiterpene derivatives is provided above.

Muscle growth may occur by increasing the fiber size and/or by increasing the number of fibers. The growth of muscles may be measured by A) increasing a wet weight, B) increasing a protein content, C) increasing the number of myofibers, and D) increasing the diameter of myofibers. An increase in myofiber growth may be defined as an increase in diameter when the diameter is defined as the minor axis of a sectional ellipsoid. A useful therapeutic agent increases the wet weight, the protein content, and/or diameter by 10% or more, more preferably 50% or more, and most preferably 100% or more in animals that had muscle degenerated by about at least 10% compared to control animals that had previously been treated similarly (i.e., animals with degenerated muscle tissue not treated with a muscle growth compound). A compound for increasing growth by increasing the number of myofibers is useful as a therapeutic agent when increasing the number of myofibers in diseased tissue by at least 1%, more preferably at least 20%, and most preferably at least 50%. These percentage values are determined relative to the basal level in untreated and disease-free comparative mammals when the compound is administered and acts locally, or in non-contralateral adult diseased muscles.

Muscle regeneration means the process by which new myofibers are formed from myoblasts. A useful therapeutic agent for regeneration increases the number of new fibers by about at least 1%, more preferably at least 20%, and most preferably, at least 50% as described above.

The differentiation of myocytes means induction of a muscle developmental program that specifies components of myofibers such as contractile organs (myofibril). A useful therapeutic agent for differentiation increases the amount of all myofibril components present in diseased tissues by about 10% or more, more preferably 50% or more, and most preferably 100% or more, compared to equivalent tissues present in similarly treated control animals.

### Health Functional Food Composition for Muscle Differentiation Promotion, Muscle Regeneration, or Muscle Strengthening

The present invention provides a health functional food composition for use in muscle differentiation promotion, muscle regeneration, or muscle strengthening, which comprises a sesquiterpene derivative or a pharmaceutically acceptable salt thereof as an active ingredient. The detailed description of the sesquiterpene derivatives is provided above.

In the health functional food composition for muscle differentiation promotion, muscle regeneration, or muscle strengthening according to the present invention, when used as an additive of a health functional food, the sesquiterpene derivative may be added to a food directly or in combination with other foods or food ingredients, and may be appropriately used using a general method. The amount of the active ingredient to be mixed may be appropriately determined according to the purpose of use such as prevention, health, treatment, or the like.

Formulations of the health functional food include not only powder, granules, pills, tablets, and capsules, but also any general foods or beverages.

The type of food is not particularly limited, and non-limiting examples of foods to which the material may be added may include meat, sausage, bread, chocolate, candies, snacks, confectionaries, pizza, ramen, other noodles, gums, dairy products including ice cream, various soups, beverages, tea, drinks, alcoholic beverages, and vitamin complexes, and may include all foods in a general sense.

Generally, for the preparation of foods or beverages, the sesquiterpene derivative may be added in an amount of 15 parts by weight or less, preferably 10 parts by weight or less, with respect to 100 parts by weight of raw materials. However, in the case of long-term ingestion for health and hygienic purposes or for health control purposes, the amount may be below the above range, and since the present invention has no safety problem because a fraction from a natural substance is used, the active ingredient may also be used in an amount above the above range.

In the health functional food according to the present invention, a beverage may include additional ingredients such as various flavoring agents, natural carbohydrates, or the like as in general beverages. Examples of the above-described natural carbohydrates include monosaccharides such as glucose and fructose, disaccharides such as maltose and sucrose, polysaccharides such as dextrin and cyclodextrin, and sugar alcohols such as xylitol, sorbitol, erythritol, and the like. As a flavoring agent, a natural flavoring agent such as a thaumatin or stevia extract, a synthetic flavoring agent such as saccharin or aspartame, or the like may be used. The proportion of the natural carbohydrates may range from about 0.01 g to 0.04 g, preferably about 0.02 g to about 0.03 g, with respect to 100 mL of the beverage according to the present invention.

In addition to the above ingredients, the health functional food composition for muscle differentiation promotion, muscle regeneration, or muscle strengthening according to the present invention may include various nutritional supplements, vitamins, electrolytes, flavors, colorants, pectic acid and salts thereof, alginic acid and salts thereof, organic acids, a protective colloid thickener, a pH adjuster, a stabilizer, a preservative, glycerin, alcohols, a carbonating agent used in carbonated beverages, and the like. In addition, the health functional food composition for muscle differentiation promotion, muscle regeneration, or muscle strengthening according to the present invention may include flesh for the preparation of natural fruit juice, fruit juice beverages, and vegetable beverages. These ingredients may be used alone or a combination thereof may be used. The proportion of these additives is not limited, but the amounts of the additives generally range from 0.01 part by weight to 0.1 part by weight with respect to 100 parts by weight of the health functional food of the present invention.

### Pharmaceutical Composition for Increasing Muscle Mass or Promoting Myogenesis

The present invention provides a pharmaceutical composition for use in increasing muscle mass or promoting myogenesis, which includes a sesquiterpene derivative or a pharmaceutically acceptable salt thereof as an active ingredient. The detailed description of the sesquiterpene derivative is provided above.

The composition according to the present invention has an effect of increasing muscle mass or promoting myogenesis. In particular, "muscle mass increase" is to improve the growth of body components, especially muscles, and may include an increase in muscle mass through physical exercise and improved endurance, and an increase in muscle mass using a method of administering, into the body, a material having an effect of increasing muscles, and the type of muscles is not limited.

In particular, according to one embodiment of the present invention, when mouse myoblasts reduced by dexamethasone were treated with cedrene, it can be confirmed that the number of myotubes of the mouse myoblasts was significantly increased. That is, the sesquiterpene derivative of the present invention may increase the thickness of myotubes in mouse myoblasts, thereby inhibiting muscle loss and promoting the growth of muscles. In addition, when mouse myoblasts reduced by dexamethasone are treated with cedrene, it can be confirmed that the sesquiterpene derivative not only significantly increases the expression of the p-4E-BP1 protein and p-p70S6K protein associated with protein synthesis, but also significantly reduces the expression of MuRF1 and Mafbx/atrogin1, which are proteins that induce muscle reduction. That is, the sesquiterpene derivative of the present invention may increase the phosphorylation of the 4E-BP1 protein and the p70S6K protein in mouse myoblasts, and may increase muscle mass by inhibiting the gene expression of MuRF1 and Mafbx/atrogin1.

According to another embodiment of the present invention, when cedrene was orally administered to mice fed a normal diet or a high-fat diet, the grip strength of the mice was significantly increased, and the time that the mice hung upside down on the cover of a rotating cage was significantly increased, from which it can be confirmed that the limb muscle strength of the mice was increased. That is, the sesquiterpene derivatives of the present invention may strengthen muscles, thereby increasing the grip strength and limb muscle strength of mice.

### Health Functional Food Composition for Increasing Muscle Mass or Promoting Myogenesis

The present invention provides a health functional food composition for use in increasing muscle mass or promoting myogenesis, which includes a sesquiterpene derivative or a pharmaceutically acceptable salt thereof as an active ingredient. The detailed description of the sesquiterpene derivatives is provided above.

### Health Functional Food Composition for Improving Muscle Function

The present invention provides a health functional food composition for use in improving muscle function, which includes a sesquiterpene derivative or a pharmaceutically acceptable salt thereof as an active ingredient. The detailed description of the sesquiterpene derivatives is provided above.

### Livestock Feed Composition for Prevention or Alleviation of Muscle Diseases, Muscle Differentiation Promotion, Muscle Regeneration, Muscle Function Improvement, or Muscle Strengthening

The present invention also provides a livestock feed composition for use in preventing or alleviating a muscle disease, which includes a sesquiterpene derivative or a pharmaceutically acceptable salt thereof as an active ingredient. The detailed description of the sesquiterpene derivatives is provided above.

The present invention also provides a livestock feed composition for use in muscle differentiation promotion, muscle regeneration, muscle function improvement, or muscle strengthening, which includes a sesquiterpene derivative or a pharmaceutically acceptable salt thereof as an active ingredient. The detailed description of the sesquiterpene derivatives is provided above.

The livestock may be one type of livestock selected from the group consisting of cows, pigs, chickens, ducks, goats, sheep, and horses, but the present invention is not limited thereto.

The feed composition may include a feed additive. The feed additive of the present invention corresponds to a feed supplement in the Feed Control Act.

As used herein, the term "feed" may refer to any natural or artificial diet, meal, or the like, or components of such meal intended or suitable to be eaten, taken in, or digested by animals.

The type of feed is not particularly limited, and any feed generally used in the art may be used. Non-limiting examples of the feed may include plant-based feed, such as grains, nuts, food by-products, seaweeds, fibers, drug by-products, fats and oils, starches, gourds, and grain by-products; and animal-based feed such as proteins, inorganic matter, fats and oils, minerals, single cell proteins, animal plankton, and foods. These may be used alone or a mixture of two or more thereof may be used.

In addition, the feed additive may further include a carrier accepted by a unit animal. In the present invention, the feed additive may be added as it is or a carrier, a stabilizer, and the like may be added thereto, and various nutrients such as vitamins, amino acids, minerals, and the like, antioxidants, other additives, and the like may be added according to need, and the form thereof may be in a suitable state such as powder, a granule, a pellet, a suspension, or the like. The feed additive of the present invention may be supplied alone or in combination with feed to a unit animal.

The present invention also provides the claimed compositions for use in a method of preventing or treating a muscle disease, promoting muscle differentiation, regenerating muscles, or strengthening muscles, including administering, to a subject, a pharmaceutical composition including a sesquiterpene derivative or a salt thereof as an active ingredient, or allowing it to be taken.

The present invention also provides the claimed compositions for use in preventing or treating a muscle disease, promoting muscle differentiation, regenerating muscles, or strengthening muscles, the composition including a sesquiterpene derivative or a salt thereof as an active ingredient.

As described above, the composition of the present invention comprising a sesquiterpene derivative or a pharmaceutically acceptable salt thereof as an active ingredient may increase the phosphorylation of the 4E-BP1 protein and the p70S6K1 protein in myoblasts and inhibit the gene expression of MuRF1 and MaFbx/atrogin1, thereby exhibiting a muscle strengthening effect through muscle differentiation, muscle regeneration, and an increase in muscle mass in muscle diseases caused by muscle dysfunction, muscle wasting, or muscle degeneration, and may inhibit muscle loss. Thus, the composition may be used to prevent or treat a muscle disease, promote muscle differentiation, regenerate muscles, and increase muscle mass or improve muscle function.

Hereinafter, exemplary examples will be described to aid in understanding of the present invention which are provided to facilitate the understanding of the present invention.

### Preparation Example 1. Cell Culture

A mouse myoblast cell line (C2C12 cells) was purchased from ATCC (Manassas, VA, USA), and the purchased cells were incubated in a 5% CO₂ incubator at 37 °C using 10% fetal bovine serum media (Gibco-BRL). When the confluency of the incubated cells became 80%, the myoblasts were allowed to differentiate into myotubes using 2% horse serum media (Gibco-BRL).

### Example 1. Treatment with Dexamethasone and Sesquiterpene Derivatives

The mouse myoblasts incubated in Preparation Example 1 were co-treated with 50 µM dexamethasone (dexa; Sigma) and 100 µM of sesquiterpene derivatives (experimental materials) for two days from day 4 of differentiation of the mouse myoblasts.

As the sesquiterpene derivatives, α-cedrene (CAS number 469-61-45, Sigma), cedrenol (CAS Number 13567-41-4, Sigma), methyl cedryl ketone (CAS Number 32388-55-9, Sigma), cedrene epoxide (CAS Number 29597-36-2, Sigma), β-cedrene (CAS number 546-28-1, Sigma), cedryl acetate (CAS Number 77-54-3, Sigma), and cedrol (CAS Number 77-53-2, Sigma) were used.

### Comparative Example 1. Dexamethasone Treatment

An experiment was conducted in the same manner as in Example 1, except for treatment with 50 µM of dexamethasone (dexa; Sigma) instead of the sesquiterpene derivatives (experimental materials).

### Experimental Example 1. Efficacy of Sesquiterpene Derivatives on Inhibiting Muscle Loss Using Mouse Myoblasts

### 1-1) Giemsa-Wright staining

The myotubes according to Example 1 and Comparative Example 1 were washed twice with phosphate buffered saline (PBS), and then fixed with 100% methanol for 10 minutes. When the fixing was completed, the myotubes were naturally dried at room temperature for 10 minutes, and then a Giemsa-Wright staining solution (Asan Pharmaceutical, Seoul), which specifically stains myotubes, was dropped onto the myotubes and left for 30 minutes to stain the myotubes.

### 1-2) Myotube Thickness Measurement

The myotubes stained in Experimental Example 1-1) were photographed using a fluorescence microscope (IX 71, Olympus) at a magnification of 20x, and then analyzed using ImageJ software (USA). Six sections were randomly selected from each well and micrographed, and the thickness of at least 1007 myotubes from each well was analyzed (three repetitions/group).

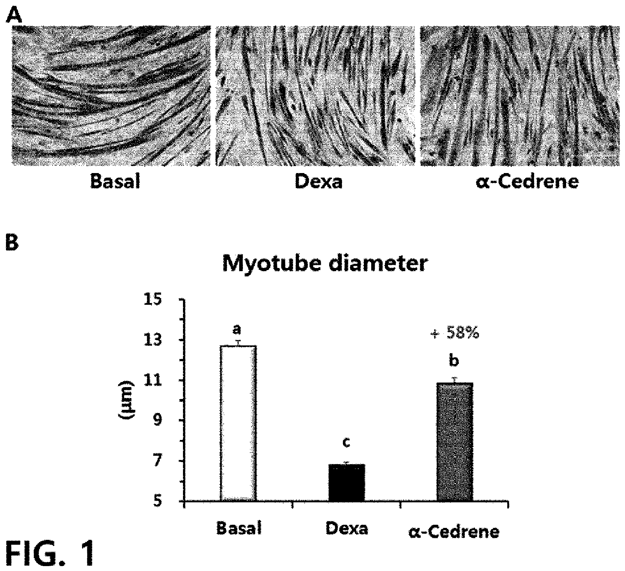

FIG. 1 illustrates changes in thickness of myotubes in mouse myoblasts.

As illustrated in FIG. 1A, it was visually confirmed that the thickness of myotubes was remarkably reduced in the case of Comparative Example 1 treated with dexamethasone (Dexa), compared to normal cells not treated with dexamethasone, and the thickness of myotubes reduced by dexamethasone was increased in the case of Example 1 treated with α-cedrene. In addition, as illustrated in FIG. 1B, from results quantified using ImageJ software, it can also be confirmed that the thickness of myotubes reduced by dexamethasone is significantly increased by 58% in the case of Example treated with cedrene.

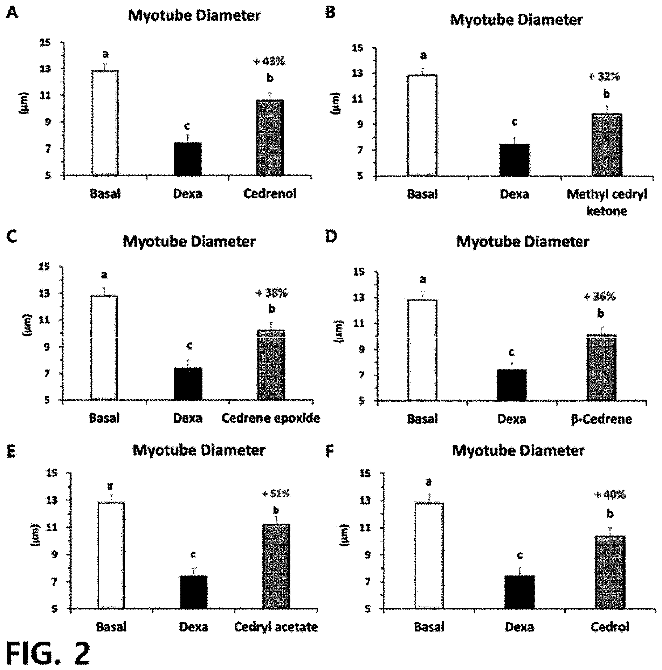

In addition, similarly, cedrenol, methyl cedryl ketone, cedrene epoxide, β-cedrene, cedryl acetate, and cedrol also significantly increased the thickness of myotubes reduced by dexamethasone by 43%, 32%, 38%, 36%, 51%, and 40%, respectively (see FIG. 2).

That is, it can be seen that the sesquiterpene derivatives including cedrene increase the thickness of myotubes in mouse myoblasts, thereby inhibiting muscle loss and promoting muscle growth.

### Experimental Example 2. Verification of Action Mechanism

### 2-1) RNA Extraction using TRIzol Method and Reverse Transcription-Polymerase Chain Reaction (RT-PCR)

334 µl of a TRIzol solution per 1 x 10⁷ mouse myoblasts of Example 1 was added and replaced, followed by centrifugation at 12,000 xg and 4 °C for 10 minutes. The supernatant was transferred to a new tube and then 67 µl of chloroform was added thereto, followed by vortexing. The supernatant was transferred again to a new tube, and isopropanol was added thereto in a ratio of 1:1 of isopropanol to supernatant, followed by vigorous shaking 10 times and being left at room temperature for 15 minutes, and centrifugation was carried out at 12,000 xg and 4 °C for 10 minutes to remove a supernatant, and 1 ml of 70% ethanol was added to the residual precipitate, followed by centrifugation at 7,500 xg and 4 °C for 5 minutes. After ethanol was removed, the tubes containing RNA precipitates were dried at room temperature for 15 minutes, and RNA pellets were dissolved using nuclease free water. The concentration of the extracted RNA samples was measured at wavelengths of 260 nm and 280 nm using a UV/VIS spectrometer (Beckman Coulter, DU730), and was subjected to agarose gel electrophoresis to confirm the integrity of RNA samples.

Reverse transcription-polymer chain reaction was performed on the RNA samples extracted from the mouse myoblasts using oligo dT primers and SuperScript Reverse Transcriptase (GIBCO BRL, Gaithersburg, MD, USA) to synthesize cDNA. The cDNA obtained by reverse transcription was used as a template and PCR was performed using, as primers, the 5' and 3' flanking sequences of cDNA to be amplified, and the used primer sequences are shown in Table 1 below. 1 µl of each amplified PCR product was subjected to electrophoresis on agarose gel to confirm generated DNA bands.

**[Table 1]**

| Gene description | SEQ NO. | Primers | Sequence (5'→3') | Annealing temperature (°C) | PCR product (bp) |
|---|---|---|---|---|---|
| MaFbx (synonym: atrogin-1) | 1 | F | GTCCAGAGAGTCGGCAAGTC | 63 | 141 |
| | 2 | R | GTCGGTGATCGTGAGACCTT | | |
| MuRF1 (synonym: TRAM63) | 3 | F | ACATCTACTGTCTCACGTGT | 58 | 106 |
| | 4 | R | TGTCCTTGGAAGATGCTTTG | | |
| Glyceraldehyde 3-phosphate dehydrogenase (GAPDH) | 5 | F | GTGATGGCATGGACTGTGGT | 55 | 163 |
| | 6 | R | GGAGCCAAAAGGGTCATCAT | | |

### 2-2) Western blotting

To perform western blotting on cells, a lysis buffer containing 500 µL of 100 mM Tris-HCl, pH 7.4, 5 mM EDTA, 50 mM sodium pyrophosphate, 50 mM NaF, 100 mM orthovanadate, 1% Triton X-100, 1 mM phenylmethanesulfonyl fluoride, 2 µg/mL of aprotinin, 1 µg/mL of pepstatin A, and 1 µg/mL of leupeptin was added to each well from which media were removed, the cells were harvested, and then centrifuged at 1,300 xg and 4 °C for 20 minutes, and then the middle layer was taken, and the concentrations of proteins were quantified by the Bradford method (Bio-Rad). 40 µg of the quantified proteins were electrophoresed by SDS-polyacrylamide gel, and then the separated proteins were transferred to nitrocellulose membranes (Amersham, Buckinghamshire, UK). Then, the membranes were washed three times for 10 minutes using a Tris-buffered saline and Tween 20 solution (TBS-T), followed by blocking using 10% skim milk for 60 minutes. Primary antibodies diluted at a ratio of 1:1,000 were added to the blocked membranes, and gently shaken at 4 °C and incubated for 12 hours, followed by washing using TBS-T, and the membranes were incubated together with secondary antibodies diluted at a ratio of 1:2,000 for 60 minutes and washed. At this time, as the primary antibodies, p70S6K1, phopho-p70S6K1 (p-p70S6K1), 4E-BP1, phospho-4E-BP1 (p-4E-BP1), and GAPDH (Cell Signaling Technology, Beverly, MA, USA) were used. Finally, the proteins were visualized on X-ray film using an ECL western blot detection kit (RPN2106, Amersham, Arlington Heights, IL, USA). Bands visualized on the X-ray film were scanned and quantified using Quantity One analysis software (Bio-Rad).

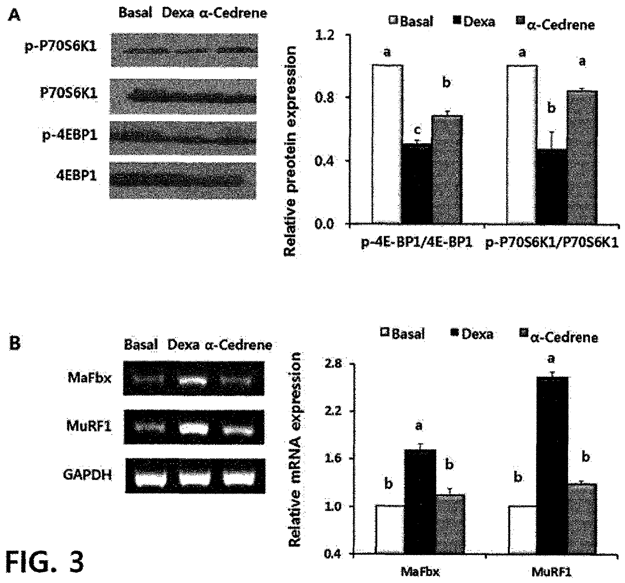

FIG. 3 is a set of graphs showing changes in expression of proteolysis- and protein synthesis-related molecules in mouse myoblasts treated with cedrene.

As illustrated in FIG. 3A, it can be confirmed that the amounts of the p-4E-BP1 protein and the p-p70S6K1 protein, which are associated with protein synthesis, were significantly reduced in the case of Comparative Example 1, compared to normal cells not treated with dexamethasone (Basal), and as illustrated in FIG. 3B, it can be confirmed that the expression of MaFbx/atrogin1 and MuRF1, which are protein degradation genes, was significantly increased. In contrast, it can be confirmed that the amounts of the p-4E-BP1 protein and the p-p70S6K protein, reduced by dexamethasone are significantly increased in the case of Example 1 treated with cedrene (see FIG. 3A), and the expression of MuRF1 and MaFbx/atrogin1 is significantly reduced (see FIG. 3B). That is, it is considered that cedrene increases the phosphorylation of the 4E-BP1 protein and the p70S6K1 protein, and inhibits the gene expression of MuRF1 and MaFbx/atrogein1, and thus is ultimately involved in increasing muscle mass.

### Preparation Example 2. Breeding of Laboratory Animals

32 5-week-old male C57BL/6N mice (Orient, Korea) were adapted to a laboratory environment for one week with a commercial normal diet (rodent chow), and then randomly divided into four groups (chow vehicle group, chow α-cedrene group, HFD vehicle group, and HFD α-cedrene group) according to a randomized block design and bred for a total of 10 weeks.

### Example 2. Normal Diet Intake and Cedrene Administration (CHOW α-cedrene group)

While mice bred in Preparation Example 2 were fed a normal diet for 10 weeks, 200 mpk α-cedrene was orally administered to the mice once a day.

### Example 3. High-Fat Diet Intake and Cedrene Administration (HFD α-cedrene group)

A procedure was carried out in the same manner as in Example 2, except that the mice were fed a high-fat experimental diet (HFD: 40% high fat energy, 17 g lard + 3% corn oil/100 g diet).

### Comparative Example 2. Normal Diet (CHOW) Intake (chow vehicle group)

A procedure was carried out in the same manner as in Example 2, except that a vehicle was orally administered instead of 200 mpk α-cedrene, while a commercial normal diet (rodent chow) was ingested.

### Comparative Example 3. High-Fat Diet (HFD) Intake (HFD vehicle group)

A procedure was carried out in the same manner as in Comparative Example 2, except that a high-fat diet (HFD: 40% high fat energy) was ingested.

### Experimental Example 3. Mouse Muscle Strength Test

### 3-1) Grip Strength Test

The grip strength of the mice of Examples 2 and 3 and Comparative Examples 2 and 3 was measured at week 12 of breeding using a grip strength meter for mice (DJ2-248, Daejong Instrument Industry Co., Ltd., Korea). In particular, a force (N) for mice to hold onto a wire mesh was measured while mounting the four feet of mice on a wire mesh to which a gauge was attached, and pulling the tails of the mice backward. The measurement was successively performed five times, and the maximum value was determined as grip strength. To normalize the experimental results, grip strength (N) divided by body weight (mg) was calculated.

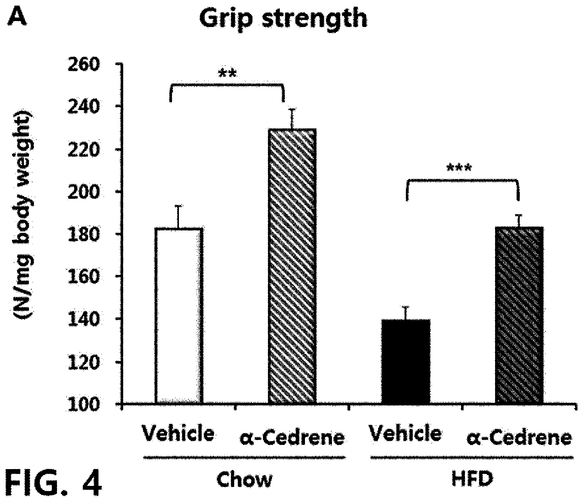

FIG. 4 illustrates the results of confirming increases in grip strength of mice by intake of cedrene.

As illustrated in FIG. 4, grip strength was significantly increased by 26% and 31% in the mice of Examples 2 and 3 fed a normal diet and a high-fat diet, respectively, compared to the case of Comparative Examples 2 and 3. That is, it can be seen that oral administration of cedrene can significantly increase the grip strength of mice regardless of the normal diet or the high-fat diet.

### 3-2) Four Limb Hanging Test

To measure the limb muscle strength of mice, the time that the mice of Examples 2 and 3 and Comparative Examples 2 and 3 hung upside down using their four feet was measured at week 12 of breeding. The time (seconds) that the mice hung upside down in a cage equipped with a wire mesh cover (diameter < 0.5 cm) (20 x 30 x 50 cm, Jeung-Do Bio & Plant Co., Ltd, Korea) was measured a total of three times, and a rest period of 30 minutes or longer was given between measurements. Experimental results were obtained by multiplying hanging time (sec) by body weight (kg).

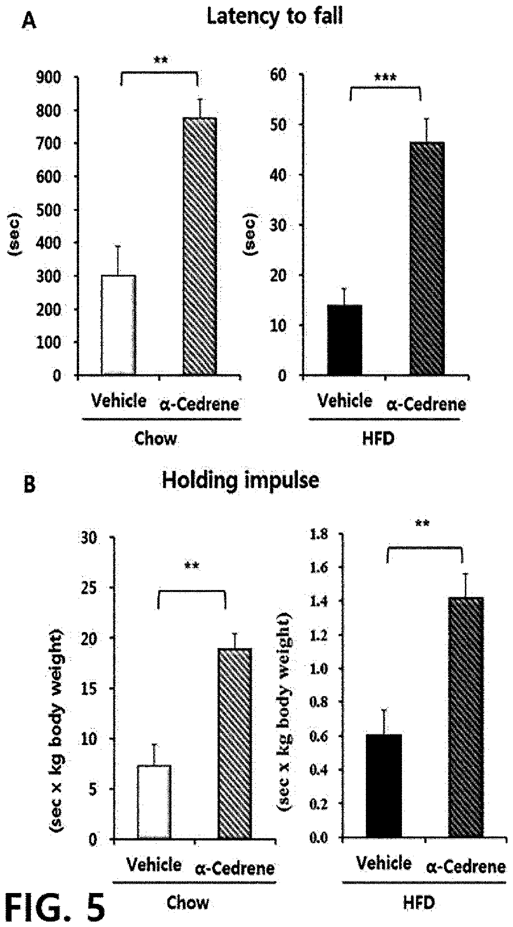

FIG. 5 illustrates the results of confirming increases in limb muscle strength of mice by cedrene intake.

As illustrated in FIG. 5A, hanging time was significantly increased in the mice of Example 2 or 3 fed a normal diet or a high-fat diet, compared to Comparative Examples 2 and 3, and as illustrated in FIG. 5B, values obtained by multiplying hanging time by body weight were also significantly increased. That is, it was confirmed that oral administration of cedrene could significantly increase the limb muscle strength of mice regardless of the normal diet or the high-fat diet.

### 3-3) Immunohistochemical Staining of Muscle Tissue

Muscle tissues of mice were extracted, fixed in 10% formalin, and then stained with hematoxylin and eosin (H&E) after requesting Korea CFC (Gyeonggi-do, Korea) to do so, and observed using an optical microscope (IX71, Olympus, JPN) and photographed using a digital camera (DP71, Olympus, JPN).

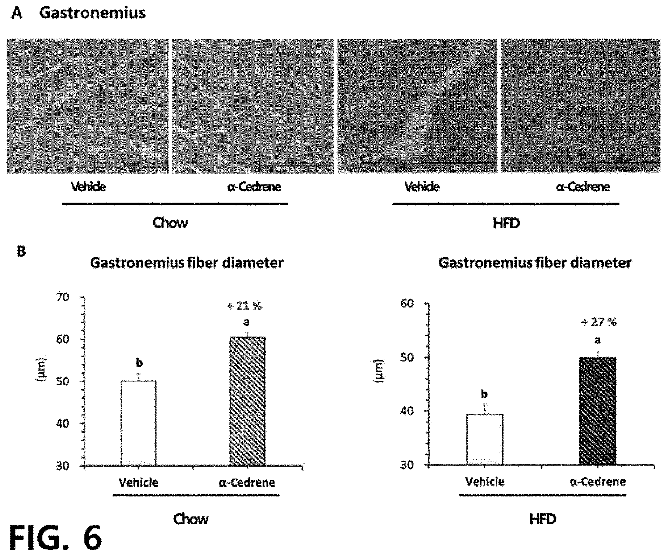

FIG. 6 illustrates the results of confirming changes in fiber diameter of muscle tissues of mice by intake of cedrene.

As illustrated in FIG. 6A, it was confirmed through microscopic observation that an increase in fiber diameter of the mouse gastrocnemius was increased by cedrene administration under both conditions of a normal diet and a high-fat diet, and it was confirmed that the increase in fiber diameter of the mouse gastrocnemius was significantly increased by 21% and 27% in the CHOW group and the HFD group, respectively (see FIG. 6B). From this result, it was confirmed that cedrene exhibited an excellent effect of increasing skeletal muscles regardless of the normal diet or the high-fat diet.

### 3-4) Statistical Analysis

The statistical analysis of all data was performed using the Statistical Package for the Social Sciences (SPSS version 21.0, IBM, Armonk, NY, USA), and analysis values were expressed as mean ± SEM. Significant differences between the examples and the comparative examples were verified by performing an independent sample T-test. When a significant difference from the vehicle group was observed, significance for the difference in average value between the groups was expressed alphabetically (a, P < 0.05; b, P < 0.01; c, P < 0.001).

### MODE OF INVENTION

Hereinafter, preparation examples of compositions including the extract of the present invention will be described which are provided for illustrative purposes.

### Preparation Example 1: Preparation of Powder

| | |
|---|---|
| Sesquiterpene derivative | 20 mg |
| Lactose hydrate | 100 mg |
| Talc | 10 mg |

The above ingredients were mixed and put into an airtight bag, thereby completing the preparation of powder.

### Preparation Example 2: Preparation of Tablets

| | |
|---|---|
| Sesquiterpene derivative | 10 mg |
| Corn starch | 100 mg |
| Lactose hydrate | 100 mg |
| Magnesium stearate | 2 mg |

The above ingredients were mixed and then tablets were prepared using a general tablet preparation method.

### Preparation Example 3. Preparation of Capsules

| | |
|---|---|
| Sesquiterpene derivative | 10 mg |
| Microcrystalline cellulose | 3 mg |
| Lactose hydrate | 14.8 mg |
| Magnesium stearate | 0.2 mg |

The above ingredients were mixed, and then gelatin capsules were filled therewith using a general capsule preparation method, thereby completing the preparation of capsules.

### Preparation Example 4: Preparation of Injections

| | |
|---|---|
| Sesquiterpene derivative | 10 mg |
| Mannitol | 180 mg |
| Sterile distilled water for injection | 2,974 mg |
| Dibasic sodium phosphate | 26 mg |

The above ingredients were mixed, and then an injection was prepared with the above-described content per 1 ampoule (2 mL) according to a general injection preparation method.

### Preparation Example 5: Preparation of Liquids

| | |
|---|---|
| Sesquiterpene derivative | 10 mg |
| Isomerized sugar | 10 mg |
| Mannitol | 5 mg |
| Purified water | appropriate amount |
| Lemon flavor | appropriate amount |

The above ingredients were added to purified water and dissolved therein according to a general liquid preparation method, and an appropriate amount of a lemon flavor was added thereto, and then purified water was added to the resulting solution to adjust a total amount to 100 mL, and then a brown vial was filled with the resulting mixture and sterilized, thereby completing the preparation of a liquid.

### Preparation Example 6: Preparation of Health Functional Food

| | |
|---|---|
| Sesquiterpene derivative | 10 mg |
| Vitamin mixture | appropriate amount |
| Vitamin A acetate | 70 µg |
| Vitamin E | 1.0 mg |
| Vitamin B₁ | 0.13 mg |
| Vitamin B₂ | 0.15 mg |
| Vitamin B₆ | 0.5 mg |
| Vitamin B₁₂ | 0.2 µg |
| Vitamin C | 10 mg |
| Biotin | 10 µg |
| Nicotin acid amide | 1.7 mg |
| Folic acid | 50 µg |
| Calcium pantothenate | 0.5 mg |
| Mineral mixture | appropriate amount |
| Ferrous sulfate | 1.75 mg |
| Zinc oxide | 0.82 mg |
| Magnesium carbonate | 25.3 mg |
| Monobasic potassium phosphate | 15 mg |
| Dibasic calcium phosphate | 55 mg |
| Potassium citrate | 30 mg |
| Calcium carbonate | 100 mg |
| Magnesium chloride | 24.8 mg |

Although ingredients relatively suitable for use in health foods are mixed in the above-described composition ratio of the vitamin and mineral mixture as an exemplary embodiment, the mixing ratio may be arbitrarily varied, and the above-listed ingredients may be mixed according to a general health food preparation method, and then prepared into granules, which may then be used for the preparation of a health food composition according to a general method.

### Preparation Example 7: Preparation of Health Drinks

| | |
|---|---|
| Sesquiterpene derivative | 10 mg |
| Vitamin C | 15 g |
| Vitamin E (powder) | 100 g |
| Iron lactate | 19.75 g |
| Zinc oxide | 3.5 g |
| Nicotinic acid amide | 3.5 g |
| Vitamin A | 0.2 g |
| Vitamin B₁ | 0.25 g |
| Vitamin B₂ | 0.3 g |
| Purified water | appropriate amount |

The above-listed ingredients are mixed according to a general method of preparing a health drink, the mixture is heated and stirred at 85°C for about 1 hour to prepare a solution, the solution is filtered, the filtrate is collected in a 2 L sterilized container and then sealed and sterilized, followed by refrigerated storage, which is then used for the preparation of a heath drink composition according to the present prevention.

Although ingredients relatively suitable for use in favorite beverages are mixed in the above-described composition ratio as an exemplary example, the mixing ratio may be arbitrarily varied depending on local and national preferences such as demand classes, demand countries, purposes of use, and the like.

Hereinafter, preparation examples of cosmetic compositions including the extract of the present invention will be described which are provided for illustrative purposes only and are not encompassed by the present invention.

### Preparation Example 1: Nourishing Face Lotion (Milk Lotion)

| | |
|---|---|
| Sesquiterpene derivative | 2.0 wt% |
| Squalane | 5.0 wt% |
| Beeswax | 4.0 wt% |
| Polysorbate 60 | 1.5 wt% |
| Sorbitan sesquioleate | 1.5 wt% |
| Liquid paraffin | 0.5 wt% |
| Caprylic/capric triglyceride | 5.0 wt% |
| Glycerin | 3.0 wt% |
| Butylene glycol | 3.0 wt% |
| Propylene glycol | 3.0 wt% |
| Carboxyvinyl polymer | 0.1 wt% |
| Triethanol amine | 0.2 wt% |
| Preservative, pigment, fragrance | appropriate amounts |
| Purified water | to 100 wt% |

Although ingredients relatively suitable for use in nourishing face lotions are mixed in the above-described composition ratio as an exemplary embodiment, the mixing ratio may be arbitrarily varied, and the nourishing face lotion may be prepared according to a general preparation method used in the cosmetic field.

### Preparation Example 2: Skin Softener (Skin Lotion)

| | |
|---|---|
| Sesquiterpene derivative | 2.0 wt% |
| Glycerin | 3.0 wt% |
| Butylene glycol | 2.0 wt% |
| Propylene glycol | 2.0 wt% |
| Carboxyvinyl polymer | 0.1 wt% |
| PEG12 nonylphenyl ether | 0.2 wt% |
| Polysorbate 80 | 0.4 wt% |
| Ethanol | 10.0 wt% |
| Triethanol amine | 0.1 wt% |
| Preservative, pigment, fragrance | appropriate amounts |
| Purified water | to 100 wt% |

Although ingredients relatively suitable for use in skin softeners are mixed in the above-described composition ratio as an exemplary embodiment, the mixing ratio may be arbitrarily varied, and the skin softener may be prepared according to a general preparation method used in the cosmetic field.

### Preparation Example 3: Nourishing Cream

| | |
|---|---|
| Sesquiterpene derivative | 2.0 wt% |
| Polysorbate 60 | 1.5 wt% |
| Sorbitan sesquioleate | 0.5 wt% |
| PEG60 hydrogenated castor oil | 2.0 wt% |
| Liquid paraffin | 10 wt% |
| Squalane | 5.0 wt% |
| Caprylic/capric triglyceride | 5.0 wt% |
| Glycerin | 5.0 wt% |
| Butylene glycol | 3.0 wt% |
| Propylene glycol | 3.0 wt% |
| Triethanol amine | 0.2 wt% |
| Preservative | appropriate amount |
| Pigment | appropriate amount |
| Fragrance | appropriate amount |
| Purified water | to 100 wt% |

Although ingredients relatively suitable for use in nourishing creams are mixed in the above-described composition ratio as an exemplary embodiment, the mixing ratio may be arbitrarily varied, and the nourishing cream may be prepared according to a general preparation method used in the cosmetic field.

### Preparation Example 4: Massage Cream

| | |
|---|---|
| Sesquiterpene derivative | 1.0 wt% |
| Beeswax | 10.0 wt% |
| Polysorbate 60 | 1.5 wt% |
| PEG60 hydrogenated castor | oil 2.0 wt% |
| Sorbitan sesquioleate | 0.8 wt% |
| Liquid paraffin | 40.0 wt% |
| Squalane | 5.0 wt% |
| Caprylic/capric triglyceride | 4.0 wt% |
| Glycerin | 5.0 wt% |
| Butylene glycol | 3.0 wt% |
| Propylene glycol | 3.0 wt% |
| Triethanol amine | 0.2 wt% |
| Preservative, pigment, fragrance | appropriate amounts |
| Purified water | to 100 wt% |

Although ingredients relatively suitable for use in massage creams are mixed in the above-described composition ratio as an exemplary embodiment, the mixing ratio may be arbitrarily varied, and the massage cream may be prepared according to a general preparation method used in the cosmetic field.

### Preparation Example 5: Pack

| | |
|---|---|
| Sesquiterpene derivative | 1.0 wt% |
| Polyvinyl alcohol | 13.0 wt% |
| Sodium carboxymethyl cellulose | 0.2 wt% |
| Glycerin | 5.0 wt% |
| Allantoin | 0.1 wt% |
| Ethanol | 6.0 wt% |
| PEG 12 nonyl phenyl ether | 0.3 wt% |
| Polysorbate 60 | 0.3 wt% |
| Preservative, pigment, flavor | appropriate amounts |
| Purified water | to 100 wt% |

Although ingredients relatively suitable for packs are mixed in the above-described mixing ratio as an exemplary example, the mixing ratio may be arbitrarily modified, and the pack may be prepared according to a general preparation method in the cosmetic field.

### Preparation Example 6: Gel

| | |
|---|---|
| Sesquiterpene derivative | 0.5 wt% |
| Sodium ethylenediamineacetate | 0.05 wt% |
| Glycerin | 5.0 wt% |
| Carboxyvinyl polymer | 0.3 wt% |
| Ethanol | 5.0 wt% |
| PEG60 hydrogenated castor oil | 0.5 wt% |
| Triethanolamine | 0.3 wt% |
| Preservative, pigment, flavor | appropriate amounts |
| Purified water | to 100 wt% |

Although ingredients relatively suitable for gels are mixed in the above-described mixing ratio as an exemplary example, the mixing ratio may be arbitrarily modified, and the gel may be prepared according to a general preparation method in the cosmetic field.

Although ingredients relatively suitable for cosmetic compositions are mixed in the above-described mixing ratio as an exemplary example, these may be applied to cosmetics for various applications including color cosmetics, and may be used in the preparation of a medicine, i.e., ointment that can be thinly applied on the human body according to the efficacy thereof, and the mixing ratio may be arbitrarily varied depending on local and national preferences such as demand classes, demand countries, purposes of use, and the like.

## Claims

1. A composition comprising a sesquiterpene derivative or a pharmaceutically acceptable salt thereof as an active ingredient for use in the prevention or the treatment of a muscle disease, wherein the sesquiterpene derivative comprises any one or more selected from compounds represented by Formulae 1 to 15 below: 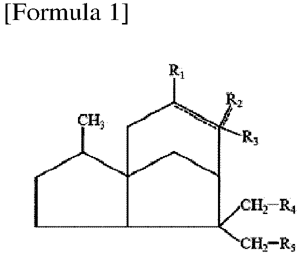 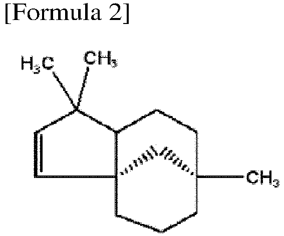 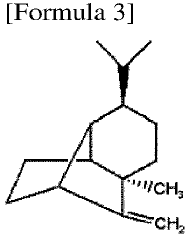 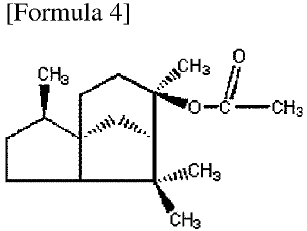 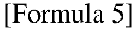 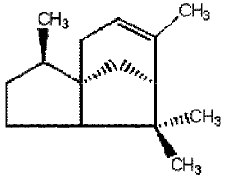 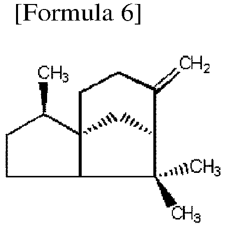 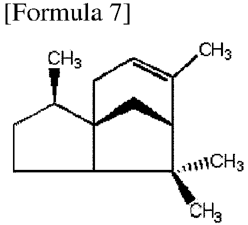 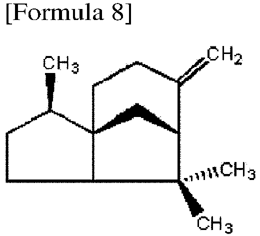 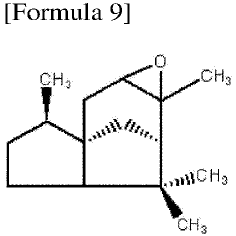 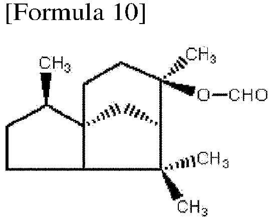 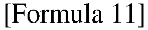 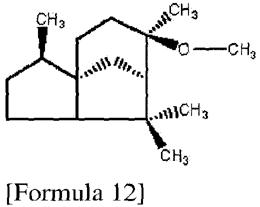 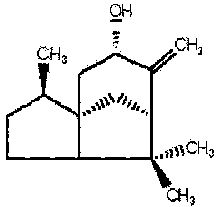 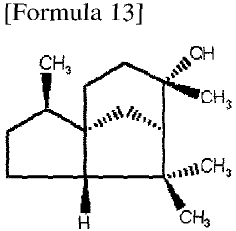 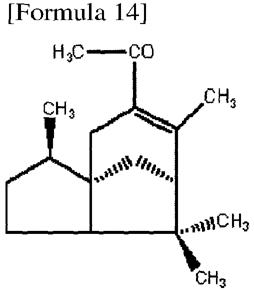 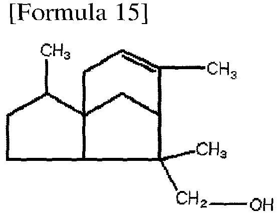 wherein, in Formula 1:
R₁ is hydrogen, hydroxyl, halo, a C₁-C₃ alkyl or -CO-R₆; R₂ is hydrogen, hydroxyl, or a C₁-C₃ alkyl; or R₁ and R₂ may be linked to each other to form an epoxide;
R₃ is hydrogen, a C₁-C₃ alkyl, -O-CO-R₇, or a C₁-C₃ alkoxy;
R₄ is hydrogen, hydroxyl, or a C₁-C₃ alkoxy;
R₅ is hydrogen, hydroxyl, or a C₁-C₃ alkoxy;
R₆ is hydrogen or a C₁-C₃ alkyl;
R₇ is hydrogen or a C₁-C₃ alkyl; 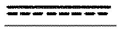
denotes a single bond or a double bond;
when 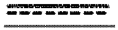 n the ring structure is a double bond, R₂ is hydrogen, hydroxyl, or a C₁-C₃ alkyl; when 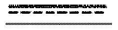 at R₂ is a double bond, R₂ is CH₂; and
when R₂ is a methyl group, R₃ is a hydroxyl group, and when R₅ is hydrogen, R₄ is not hydrogen.

2. The composition for use according to claim 1, wherein the sesquiterpene derivative is α-cedrene represented by Formula 5 below:

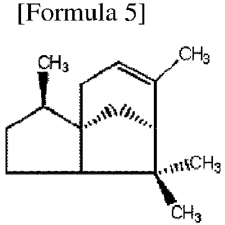

3. The composition for use according to claim 1, wherein the composition increases the expression of p-4E-BP1 and p-p70S6K proteins.

4. The composition for use according to claim 1, wherein the composition reduces the expression of muscle RING-finger protein-1 (MuRF1), muscle atrophy F-box (MaFbx), or myostatin.

5. The composition for use according to claim 1, wherein the muscle disease is a muscle disease caused by muscle dysfunction, muscle loss, muscle atrophy, muscle wasting, or muscle degeneration.

6. The composition for use according to claim 1, wherein the muscle disease comprises any one or more selected from the group consisting of atony, muscular atrophy, muscular dystrophy, myasthenia, cachexia, rigid spine syndrome, amyotrophic lateral sclerosis (Lou Gehrig's disease), Charcot- Marie- Tooth disease, and sarcopenia.

7. The composition for use according to claim 1, wherein the composition is a pharmaceutical composition, a health functional food composition or a livestock feed composition.

8. A composition comprising a sesquiterpene derivative or a pharmaceutically acceptable salt thereof as an active ingredient for use in the promotion of muscle differentiation, regeneration of muscle, improvement of muscle function, or strengthening of muscle-;
wherein the sesquiterpene derivative comprises any one or more selected from compounds represented by Formulae 1 to 15 below: 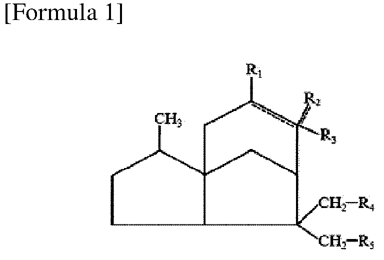 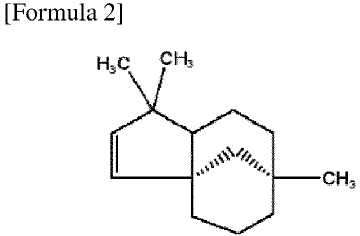 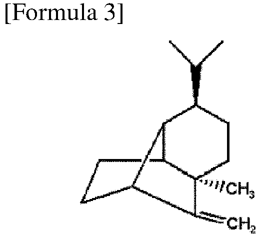 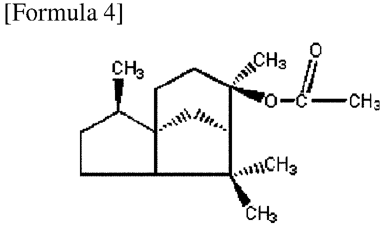 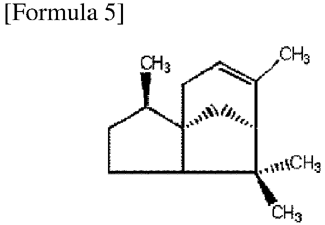 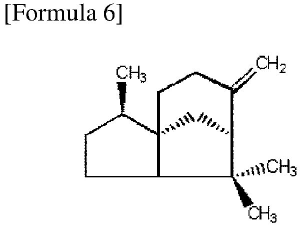 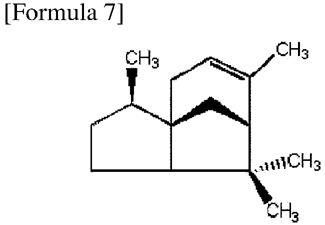 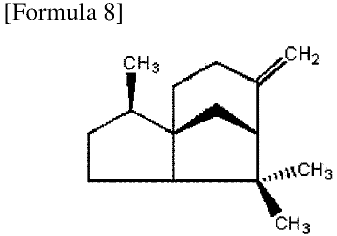 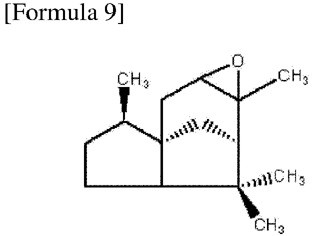 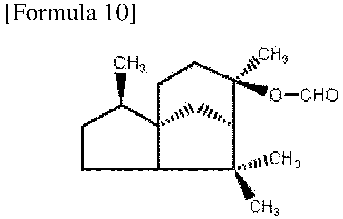 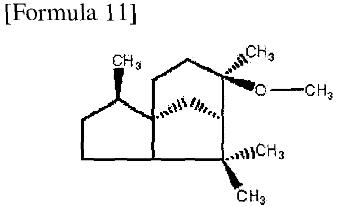 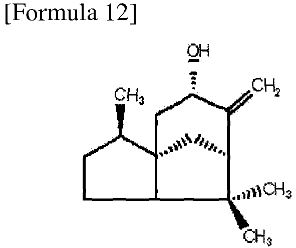 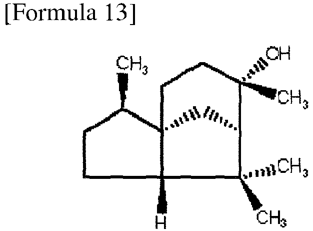 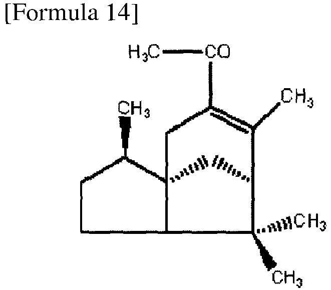 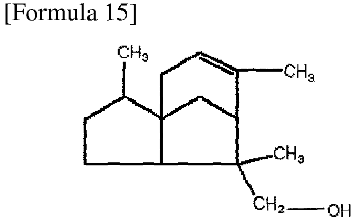
wherein, in Formulae 1:
R₁ is hydrogen, hydroxyl, halo, a C₁-C₃ alkyl or -CO-R₆; R₂ is hydrogen, hydroxyl, or a C₁-C₃ alkyl; or R₁ and R₂ may be linked to each other to form an epoxide;
R₃ is hydrogen, a C₁-C₃ alkyl, -O-CO-R₇, or a C1-C3 alkoxy;
R₄ is hydrogen, hydroxyl, or a C₁-C₃ alkoxy;
R₅ is hydrogen, hydroxyl, or a C₁-C₃ alkoxy;
R₆ is hydrogen or a C₁-C₃ alkyl;
R₇ is hydrogen or a C₁-C₃ alkyl;
-̅ -̅ -̅ -̅ -̅ -̅ -̅ -̅ denotes a single bond or a double bond; when -̅ -̅ -̅ -̅ -̅ -̅ -̅ -̅ in the ring structure is a double bond, R₂ is hydrogen, hydroxyl, or a C₁-C₃ alkyl; when -̅ -̅ -̅ -̅ -̅ -̅ -̅ -̅ at R₂ is a double bond, R₂ is CH₂; and
when R₂ is a methyl group, R₃ is a hydroxyl group, and when R₅ is hydrogen, R₄ is not hydrogen.

9. The composition for use according to claim 8, wherein the composition is a pharmaceutical composition, a health functional food composition or a livestock feed composition.

## Patentansprüche

1. Zusammensetzung, umfassend ein Sesquiterpen-Derivat oder ein pharmazeutisch annehmbares Salz davon als Wirkstoff zur Verwendung bei der Vorbeugung oder der Behandlung einer Muskelerkrankung, wobei das Sesquiterpen-Derivat eines oder mehrere umfasst, die ausgewählt sind aus den nachstehenden Formeln 1 bis 15: 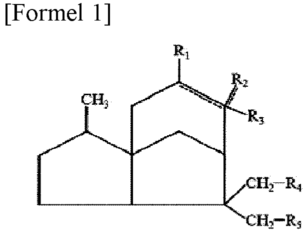 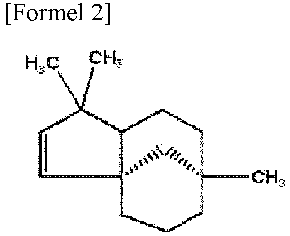 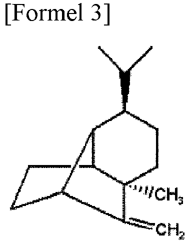 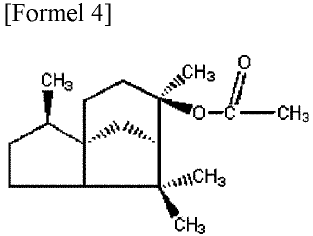 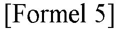 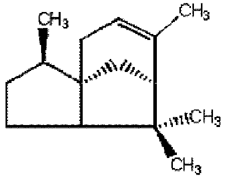 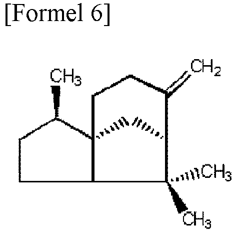 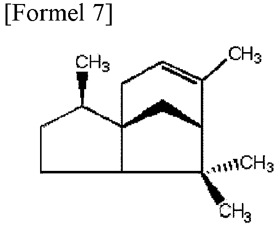 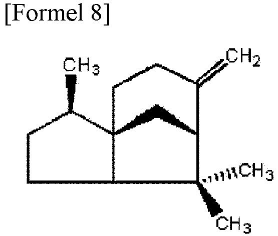 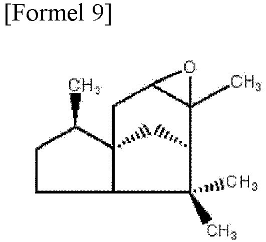 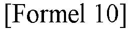 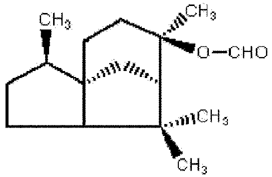 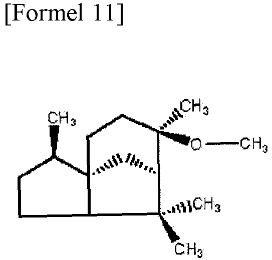 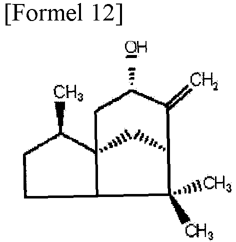 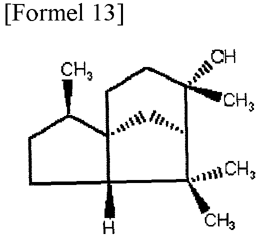 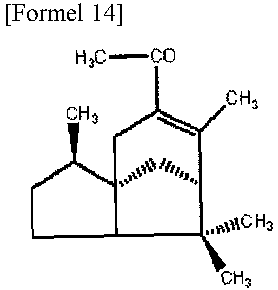 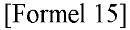 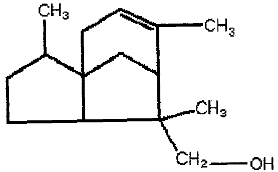 wobei in Formel 1:
R₁ Wasserstoff, Hydroxyl, Halogen, ein C₁-C₃-Alkyl oder -CO-R₆ ist; R₂ Wasserstoff, Hydroxyl, oder ein C₁-C₃-Alkyl ist; oder R₁ und R₂ miteinander verbunden sein können, um ein Epoxid zu bilden;
R₃ Wasserstoff, ein C₁-C₃-Alkyl, -O-CO-R₇ oder ein C₁-C₃-Alkoxy ist;
R₄ Wasserstoff, Hydroxyl oder ein C₁-C₃-Alkoxy ist;
R₅ Wasserstoff, Hydroxyl oder ein C₁-C₃-Alkoxy ist;
R₆ Wasserstoff oder ein C₁-C₃-Alkyl ist;
R₇ Wasserstoff oder ein C₁-C₃-Alkyl ist; 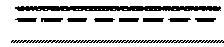 - eine Einfachbindung oder eine Doppelbindung bezeichnet;
wenn 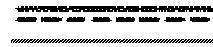 - in der Ringstruktur eine Doppelbindung ist, R₂ Wasserstoff, Hydroxyl oder ein C₁-C₃-Alkyl ist; wenn 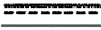 an R₂ eine Doppelbindung ist, R₂ CH₂ ist; und
wenn R₂ eine Methylgruppe ist, R₃ eine Hydroxylgruppe ist, und wenn R₅ Wasserstoff ist, R₄ nicht Wasserstoff ist.

2. Zusammensetzung zur Verwendung nach Anspruch 1, wobei das Sesquiterpen-Derivat α-Cedren ist, das durch die nachstehende Formel 5 dargestellt ist:

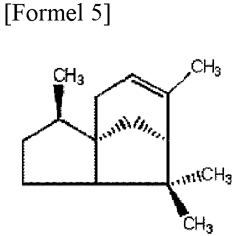

3. Zusammensetzung zur Verwendung nach Anspruch 1, wobei die Zusammensetzung die Expression der Proteine p-4E-BP1 und p-p70S6K erhöht.

4. Zusammensetzung zur Verwendung nach Anspruch 1, wobei die Zusammensetzung die Expression von Muskel-RING-Fingerprotein-1 (MuRF1), Muskelatrophie-F-Box (MaFbx) oder Myostatin reduziert.

5. Zusammensetzung zur Verwendung nach Anspruch 1, wobei die Muskelerkrankung eine Muskelerkrankung ist, die durch Muskelfunktionsstörung, Muskelverlust, Muskelatrophie, Muskelschwund oder Muskeldegeneration verursacht wird.

6. Zusammensetzung zur Verwendung nach Anspruch 1, wobei die Muskelerkrankung eine oder mehrere Erkrankungen umfasst, die ausgewählt sind aus der Gruppe, bestehend aus Atonie, Muskelatrophie, Muskeldystrophie, Myasthenie, Kachexie, Syndrom der starren Wirbelsäule, amyotropher Lateralsklerose (Lou Gehrig-Krankheit), Charcot-Marie-Tooth-Krankheit und Sarkopenie.

7. Zusammensetzung zur Verwendung nach Anspruch 1, wobei die Zusammensetzung eine pharmazeutische Zusammensetzung, eine gesundheitsfördernde funktionelle Lebensmittelzusammensetzung oder eine Viehfutterzusammensetzung ist.

8. Zusammensetzung, umfassend ein Sesquiterpen-Derivat oder ein pharmazeutisches annehmbares Salz davon als Wirkstoff zur Verwendung beim Fördern von Muskeldifferenzierung, Muskelregeneration, einer Verbesserung der Muskelfunktion oder Muskelstärkung-;
wobei das Sesquiterpen-Derivat eine oder mehrere Verbindungen umfasst, die ausgewählt sind aus den nachstehenden Formeln 1 bis 15: 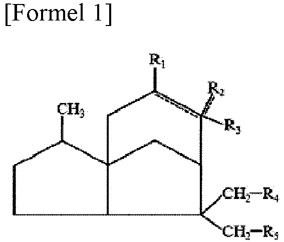 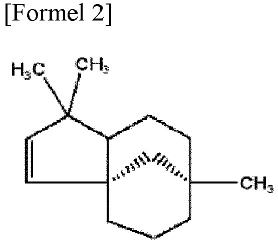 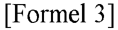 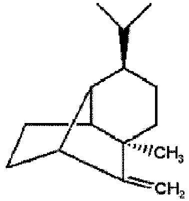 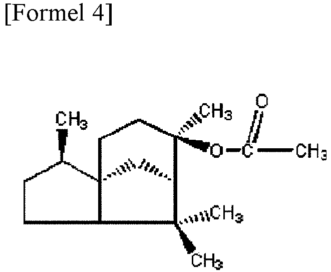 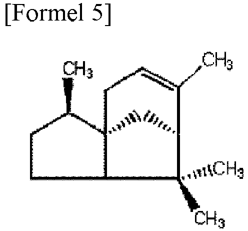 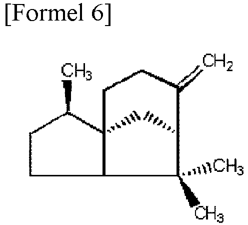 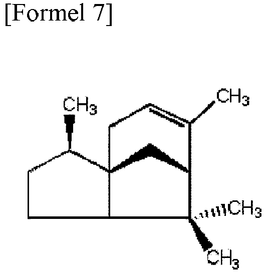 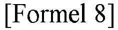 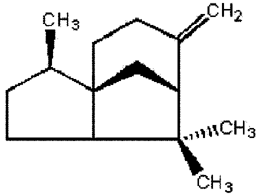 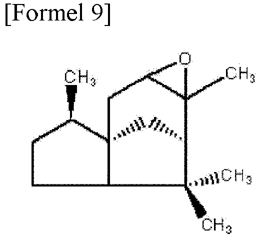 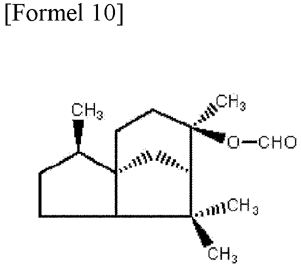 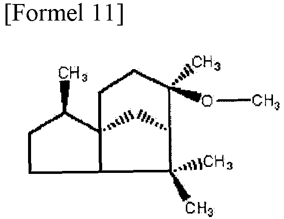 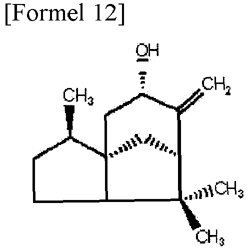 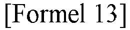 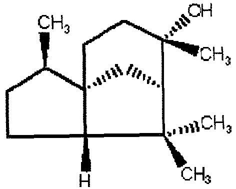 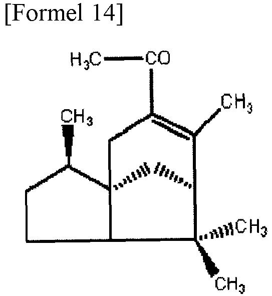 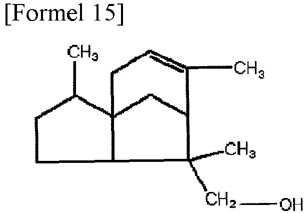
wobei in Formel 1:
R₁ Wasserstoff, Hydroxyl, Halogen, ein C₁-C₃-Alkyl oder -CO-R₆ ist; R₂ Wasserstoff, Hydroxyl, oder ein C₁-C₃-Alkyl ist; oder R₁ und R₂ miteinander verbunden sein können, um ein Epoxid zu bilden;
R₃ Wasserstoff, ein C₁-C₃-Alkyl, -O-CO-R₇ oder ein C1-C3-Alkoxy ist;
R₄ Wasserstoff, Hydroxyl oder ein C₁-C₃-Alkoxy ist;
R₅ Wasserstoff, Hydroxyl oder ein C₁-C₃-Alkoxy ist;
R₆ Wasserstoff oder ein C₁-C₃-Alkyl ist;
R₇ Wasserstoff oder ein C₁-C₃-Alkyl ist;
-̅ -̅ -̅ -̅ -̅ -̅ -̅ -̅ eine Einfachbindung oder eine Doppelbindung bezeichnet; wenn -̅ -̅ -̅ -̅ -̅ -̅ -̅ -̅ in der Ringstruktur eine Doppelbindung ist, R₂ Wasserstoff, Hydroxyl oder ein C₁-C₃-Alkyl ist; wenn -̅ -̅ -̅ -̅ -̅ -̅ -̅ -̅ an R₂ eine Doppelbindung ist, R₂ CH₂ ist; und
wenn R₂ eine Methylgruppe ist, R₃ eine Hydroxylgruppe ist, und wenn R₅ Wasserstoff ist, R₄ nicht Wasserstoff ist.

9. Zusammensetzung zur Verwendung nach Anspruch 8, wobei die Zusammensetzung eine pharmazeutische Zusammensetzung, eine gesundheitsfunktionelle Lebensmittelzusammensetzung oder eine Viehfutterzusammensetzung ist.

## Revendications

1. Composition comprenant un dérivé de sesquiterpène ou un sel pharmaceutiquement acceptable de celui-ci en tant qu'ingrédient actif destinée à être utilisée dans la prévention ou le traitement d'une maladie musculaire, ledit dérivé de sesquiterpène comprenant l'un quelconque ou plusieurs composés choisis parmi les composés représentés par les formules 1 à 15 ci-dessous : 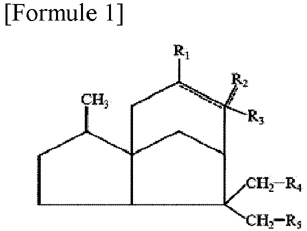 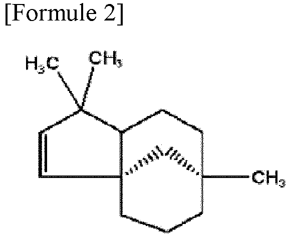 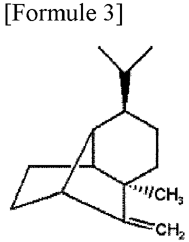 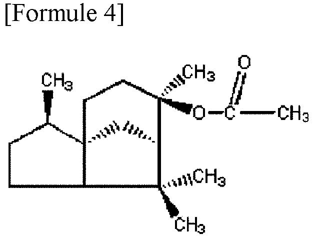 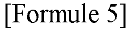 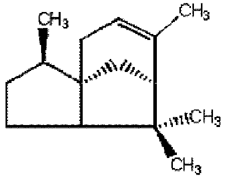 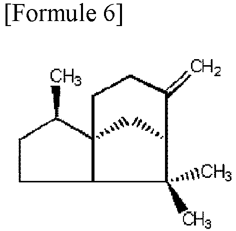 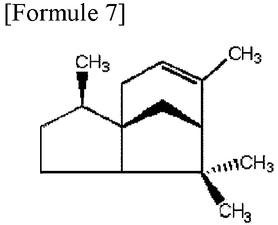 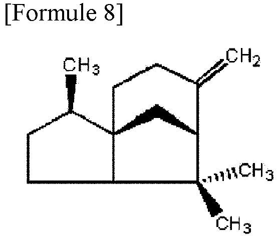 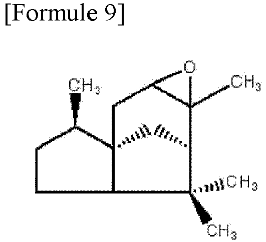 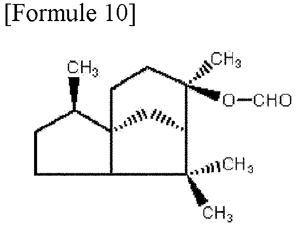 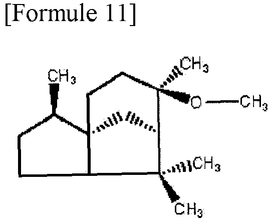 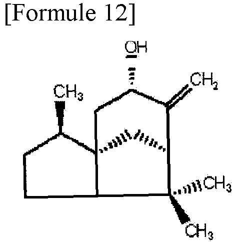 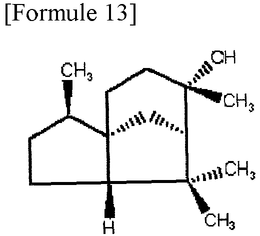 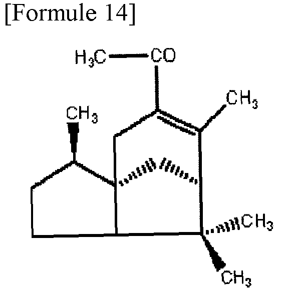 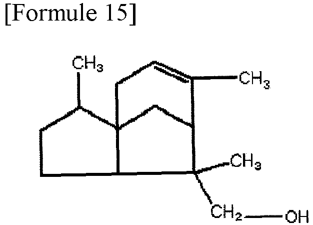 dans lesquelles, dans la formule 1 :
R₁ représente un atome d'hydrogène, un groupe hydroxy, halogéno, C₁-C₃ alkyle ou -CO-R₆ ;
R₂ représente un atome d'hydrogène, un groupe hydroxy ou C₁-C₃ alkyle ; ou
R₁ et R₂ peuvent être liés l'un à l'autre pour former un époxyde ;
R₃ représente un atome d'hydrogène, un groupe C₁-C₃ alkyle, -O-CO-R₇ ou C₁-C₃ alcoxy ;
R₄ représente un atome d'hydrogène, un groupe hydroxy ou C₁-C₃ alcoxy ;
R₅ représente un atome d'hydrogène, un groupe hydroxy ou C₁-C₃ alcoxy ;
R₆ représente un atome d'hydrogène ou un groupe C₁-C₃ alkyle ;
R₇ représente un atome d'hydrogène ou un groupe C₁-C₃ alkyle ; 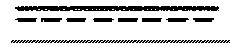
désigne une liaison simple ou une double liaison ;
lorsque 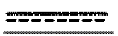 dans la structure de noyau représente une double liaison, R₂ représente un atome d'hydrogène, un groupe hydroxy ou C₁-C₃ alkyle ;
lorsque 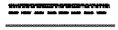 au niveau de R₂ représente une double liaison, R₂ représente un groupe CH₂ ; et
lorsque R₂ représente un groupe méthyle, R₃ représente un groupe hydroxy, et lorsque R₅ représente un atome d'hydrogène, R₄ ne représente pas un atome d'hydrogène.

2. Composition destinée à être utilisée selon la revendication 1, ledit dérivé de sesquiterpène étant le α-cédrène représenté par la formule 5 ci-dessous :

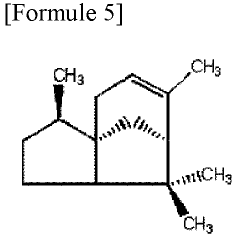

3. Composition destinée à être utilisée selon la revendication 1, ladite composition comprenant augmentant l'expression des protéines p-4E-BP1 et p-p70S6K.

4. Composition destinée à être utilisée selon la revendication 1, ladite composition réduisant l'expression de la MuRF1 (muscle RING-finger protein-1), de la MaFbx (muscle atrophy F-box) ou de la myostatine.

5. Composition destinée à être utilisée selon la revendication 1, ladite maladie musculaire étant une maladie musculaire causée par un dysfonctionnement musculaire, une perte musculaire, une atrophie musculaire, une fonte musculaire ou une dégénérescence musculaire.

6. Composition destinée à être utilisée selon la revendication 1, ladite maladie musculaire comprenant l'une quelconque ou plusieurs maladies choisies dans le groupe constitué par une atonie, une atrophie musculaire, une dystrophie musculaire, une myasthénie, une cachexie, le syndrome de la colonne vertébrale raide, la sclérose latérale amyotrophique (maladie de Lou Gehrig), la maladie de Charcot-Marie-Tooth et la sarcopénie.

7. Composition destinée à être utilisée selon la revendication 1, ladite composition étant une composition pharmaceutique, une composition d'aliments fonctionnels de santé ou une composition d'aliments pour bétail.

8. Composition comprenant un dérivé de sesquiterpène ou un sel pharmaceutiquement acceptable de celui-ci en tant qu'ingrédient actif destinée à être utilisée dans la promotion de la différenciation musculaire, la régénération musculaire, l'amélioration du fonctionnement des muscles ou le renforcement des muscles ;
ledit dérivé de sesquiterpène comprenant l'un quelconque ou plusieurs composés choisis parmi les composés représentés par les formules 1 à 15 ci-dessous : 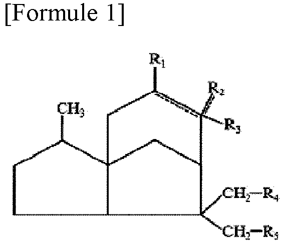 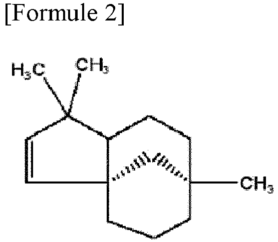 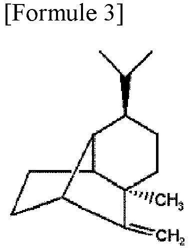 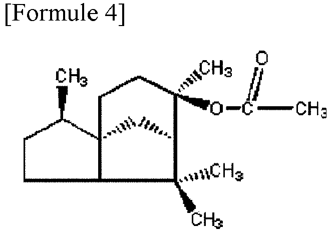 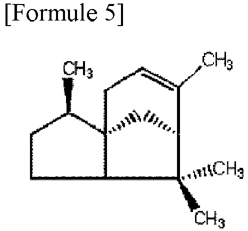 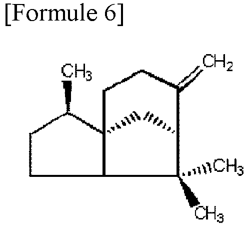 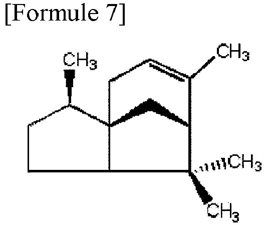 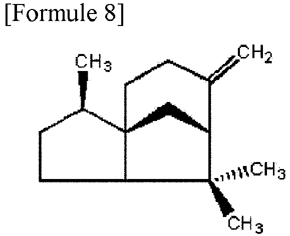 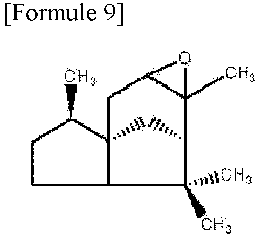 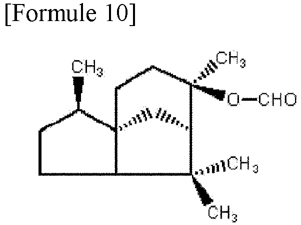 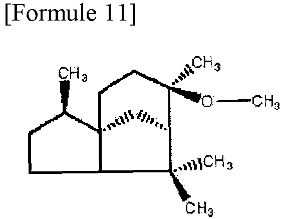 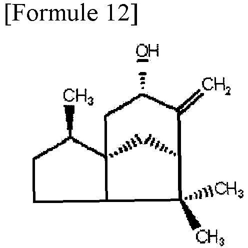 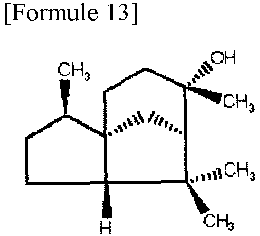 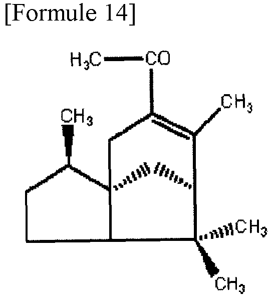 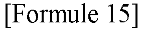 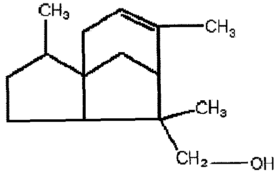
dans lesquelles, dans la formule 1 :
R₁ représente un atome d'hydrogène, un groupe hydroxy, halogéno, C₁-C₃ alkyle ou -CO-R₆ ;
R₂ représente un atome d'hydrogène, un groupe hydroxy ou C₁-C₃ alkyle ; ou
R₁ et R₂ peuvent être liés l'un à l'autre pour former un époxyde ;
R₃ représente un atome d'hydrogène, un groupe C₁-C₃ alkyle, -O-CO-R₇ ou C1-C3 alcoxy ;
R₄ représente un atome d'hydrogène, un groupe hydroxy ou C₁-C₃ alcoxy ;
R₅ représente un atome d'hydrogène, un groupe hydroxy ou C₁-C₃ alcoxy ;
R₆ représente un atome d'hydrogène ou un groupe C₁-C₃ alkyle ;
R₇ représente un atome d'hydrogène ou un groupe C₁-C₃ alkyle ; 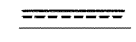
désigne une liaison simple ou une double liaison ;
lorsque 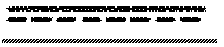 dans la structure de noyau représente une double liaison, R₂ représente un atome d'hydrogène, un groupe hydroxy ou C₁-C₃ alkyle ;
lorsque 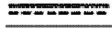 au niveau de R₂ représente une double liaison, R₂ représente un groupe CH₂ ; et
lorsque R₂ représente un groupe méthyle, R₃ représente un groupe hydroxy, et lorsque R₅ représente un atome d'hydrogène, R₄ ne représente pas un atome d'hydrogène.

9. Composition destinée à être utilisée selon la revendication 8, ladite composition étant une composition pharmaceutique, une composition d'aliments fonctionnels de santé ou une composition d'aliments pour bétail.
